# EUROPEAN PATENT SPECIFICATION

(11) **EP 2 717 941 B1**
(45) Date of publication and mention of the grant of the patent: **01.04.2020**
(21) Application number: 12727916.4
(22) Date of filing: 13.06.2012
(51) Int. Cl.: A61M 1/36, B01J 20/32

(54) **TREATING CANCER**
BEHANDLUNG VON KREBS
TRAITEMENT DU CANCER

(30) Priority: 13.06.2011 US 201161496242 P
(43) Date of publication of application: 16.04.2014
(73) Proprietor: TLA Targeted Immunotherapies AB, 171 76 Stockholm (SE)
(72) Inventor: COTTON, Graham, Edinburgh EH13 9PH (GB); WINQVIST, Ola, S-756 53 Uppsala (SE)
(74) Representative: Zacco Denmark A/S
(86) International application number: PCT/GB2012/051351
(87) International publication number: WO 2012/172341

(56) References cited:
- EP-A1- 2 067 495
- WO-A1-2012/112724
- WO-A2-2010/029317
- US-A1- 2013 108 640
- , 1 January 2010 (2010-01-01), XP055036910, Retrieved from the Internet: URL:http://www.jeccr.com/content/pdf/1756- 9966-29-16.pdf [retrieved on 2012-08-31]
- IWAO TAKANAMI: "Overexpression of CCR7 mRNA in nonsmall cell lung cancer: Correlation with lymph node metastasis", INTERNATIONAL JOURNAL OF CANCER, vol. 105, no. 2, 10 June 2003 (2003-06-10) , pages 186-189, XP055037030, ISSN: 0020-7136, DOI: 10.1002/ijc.11063
- Hitoshi Hasegawa ET AL: "Increased chemokine receptor CCR7/EBI1 expression enhances the infiltration of lymphoid organs by adult T-cell leukemia cells", , 1 January 2000 (2000-01-01), XP55445668, Retrieved from the Internet: URL:http://www.bloodjournal.org/content/bl oodjournal/95/1/30.full.pdf [retrieved on 2018-01-29]
- Chao Yan ET AL: "Expression of vascular endothelial growth factor C and chemokine receptor CCR7 in gastric carcinoma and their values in predicting lymph node metastasis", World Journal of Gastroenterology, vol. 10, no. 6, 1 January 2004 (2004-01-01), page 783, XP55445674, CN ISSN: 1007-9327, DOI: 10.3748/wjg.v10.i6.783
- LAZENNEC G ET AL: "Chemokines and chemokine receptors: new insights into cancer-related inflammation", TRENDS IN MOLECULAR MEDICINE, ELSEVIER CURRENT TRENDS, GB, vol. 16, no. 3, 1 March 2010 (2010-03-01), pages 133-144, XP026963582, ISSN: 1471-4914 [retrieved on 2010-02-15]

<110> ITH Immune Therapy Holdings AB
<120> TREATING CANCER
<130> P117755WO00
<160> 34
<170> PatentIn version 3.5
<210> 1
   <211> 76
   <212> PRT
   <213> Artificial sequence
<220>
   <223> Synthetic peptide
<220>
   <221> X
   <222> (1)..(1)
   <223> X is pyroglutamine or Gln
<220>
   <221> X
   <222> (75)..(75)
   <223> X is an amino acid residue that can be biotinylated, such as lysine, ornithine or diaminopropionic acid and optionally is biotinylated, optionally via a spacer molecule such as PEG, in particular K(PEG-Biotin)
<400> 1
   Xaa Pro Asp Ser Val Ser Ile Pro Ile Thr Cys Cys Phe Asn Val Ile
   1               5                   10                  15
   Asn Arg Lys Ile Pro Ile Gln Arg Leu Glu Ser Tyr Thr Arg Ile Thr
               20                  25                  30
   Asn Ile Gln Cys Pro Lys Glu Ala Val Ile Phe Lys Thr Lys Arg Gly
           35                  40                  45
   Lys Glu Val Cys Ala Asp Pro Lys Glu Arg Trp Val Arg Asp Ser Met
       50                  55                  60
   Lys His Leu Asp Gln Ile Phe Gln Asn Leu Xaa Pro
   65                  70                  75
<210> 2
   <211> 76
   <212> PRT
   <213> Artificial sequence
<220>
   <223> Synthetic peptide
<220>
   <221> X
   <222> (1)..(1)
   <223> X is pyroglutamine or Gln
<220>
   <221> X
   <222> (75)..(75)
   <223> X is FmocLys(ivDde)
<400> 2
   Xaa Pro Asp Ser Val Ser Ile Pro Ile Thr Cys Cys Phe Asn Val Ile
   1               5                   10                  15
   Asn Arg Lys Ile Pro Ile Gln Arg Leu Glu Ser Tyr Thr Arg Ile Thr
               20                  25                  30
   Asn Ile Gln Cys Pro Lys Glu Ala Val Ile Phe Lys Thr Lys Arg Gly
           35                  40                  45
   Lys Glu Val Cys Ala Asp Pro Lys Glu Arg Trp Val Arg Asp Ser Met
       50                  55                  60
   Lys His Leu Asp Gln Ile Phe Gln Asn Leu Xaa Pro
   65                  70                  75
<210> 3
   <211> 76
   <212> PRT
   <213> Artificial sequence
<220>
   <223> Synthetic peptide
<220>
   <221> X
   <222> (1)..(1)
   <223> X is pyroglutamine
<220>
   <221> X
   <222> (1)..(1)
   <223> X is pyroglutamine or Gln
<220>
   <221> X
   <222> (75)..(75)
   <223> X is K(PEG-Biotin)
<400> 3
   Xaa Pro Asp Ser Val Ser Ile Pro Ile Thr Cys Cys Phe Asn Val Ile
   1               5                   10                  15
   Asn Arg Lys Ile Pro Ile Gln Arg Leu Glu Ser Tyr Thr Arg Ile Thr
               20                  25                  30
   Asn Ile Gln Cys Pro Lys Glu Ala Val Ile Phe Lys Thr Lys Arg Gly
   35 40 45
   Lys Glu Val Cys Ala Asp Pro Lys Glu Arg Trp Val Arg Asp Ser Met
   50 55 60
   Lys His Leu Asp Gln Ile Phe Gln Asn Leu Xaa Pro
   65 70 75
<210> 4
   <211> 68
   <212> PRT
   <213> Artificial sequence
<220>
   <223> Synthetic peptide
<400> 4
   Ser Pro Tyr Ser Ser Asp Thr Thr Pro Cys Cys Phe Ala Tyr Ile Ala
   1 5 10 15
   Arg Pro Leu Pro Arg Ala His Ile Lys Glu Tyr Phe Tyr Thr Ser Gly
   20 25 30
   Lys Cys Ser Asn Pro Ala Val Val Phe Val Thr Arg Lys Asn Arg Gln
   35 40 45
   Val Cys Ala Asn Pro Glu Lys Lys Trp Val Arg Glu Tyr Ile Asn Ser
   50 55 60
   Leu Glu Lys Ser
   65
<210> 5
   <211> 68
   <212> PRT
   <213> Artificial sequence
<220>
   <223> Synthetic peptide
<220>
   <221> X
   <222> (67)..(67)
   <223> X is FmocLys(ivDde)
<400> 5
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
           35                  40                  45
   Val Cys Ala Asn Pro Glu Lys Lys Trp Val Arg Glu Tyr Ile Asn Ser
       50                  55                  60
   Leu Glu Xaa Ser
   65
<210> 6
   <211> 68
   <212> PRT
   <213> Artificial sequence
<220>
   <223> Synthetic peptide
<220>
   <221> X
   <222> (67)..(67)
   <223> X is an amino acid residue that can be biotinylated, such as lysine, ornithine or diaminopropionic acid and optionally is biotinylated (e.g. K-biotin), optionally via a spacer molecule such as PEG, in particular K(PEG-Biotin)
<400> 6
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
           35                  40                  45
   Val Cys Ala Asn Pro Glu Lys Lys Trp Val Arg Glu Tyr Ile Asn Ser
       50                  55                  60
   Leu Glu Xaa Ser
   65
<210> 7
   <211> 70
   <212> PRT
   <213> Artificial sequence
<220>
   <223> Synthetic peptide
<400> 7
   Ala Ser Asn Phe Asp Cys Cys Leu Gly Tyr Thr Asp Arg Ile Leu His
   1               5                   10                  15
   Pro Lys Phe Ile Val Gly Phe Thr Arg Gln Leu Ala Asn Glu Gly Cys
               20                  25                  30
   Asp Ile Asn Ala Ile Ile Phe His Thr Lys Lys Lys Leu Ser Val Cys
           35                  40                  45
   Ala Asn Pro Lys Gln Thr Trp Val Lys Tyr Ile Val Arg Leu Leu Ser
       50                  55                  60
   Lys Lys Val Lys Asn Met
   65                  70
<210> 8
   <211> 70
   <212> PRT
   <213> Artificial sequence
<220>
   <223> Synthetic peptide
<220>
   <221> X
   <222> (68)..(68)
   <223> X is FmocLys(ivDde)
<400> 8
   Ala Ser Asn Phe Asp Cys Cys Leu Gly Tyr Thr Asp Arg Ile Leu His
   1               5                   10                  15
   Pro Lys Phe Ile Val Gly Phe Thr Arg Gln Leu Ala Asn Glu Gly Cys
               20                  25                  30
   Asp Ile Asn Ala Ile Ile Phe His Thr Lys Lys Lys Leu Ser Val Cys
           35                  40                  45
   Ala Asn Pro Lys Gln Thr Trp Val Lys Tyr Ile Val Arg Leu Leu Ser
       50                  55                  60
   Lys Lys Val Xaa Asn Met
   65                  70
<210> 9
   <211> 70
   <212> PRT
   <213> Artificial sequence
<220>
   <223> Synthetic peptide
<220>
   <221> X
   <222> (68).. (68)
   <223> X is an amino acid residue that can be biotinylated, such as lysine, ornithine or diaminopropionic acid and optionally is biotinylated, optionally via a spacer molecule such as PEG, e.g. K(PEG-Biotin)
<400> 9
   Ala Ser Asn Phe Asp Cys Cys Leu Gly Tyr Thr Asp Arg Ile Leu His
   1               5                   10                  15
   Pro Lys Phe Ile Val Gly Phe Thr Arg Gln Leu Ala Asn Glu Gly Cys
               20                  25                  30
   Asp Ile Asn Ala Ile Ile Phe His Thr Lys Lys Lys Leu Ser Val Cys
           35                  40                  45
   Ala Asn Pro Lys Gln Thr Trp Val Lys Tyr Ile Val Arg Leu Leu Ser
       50                  55                  60
   Lys Lys Val Xaa Asn Met
   65                  70
<210> 10
   <211> 67
   <212> PRT
   <213> Artificial sequence
<220>
   <223> Synthesised peptide
<400> 10
   Lys Pro Val Ser Leu Ser Tyr Arg Cys Pro Cys Arg Phe Phe Glu Ser
   1               5                   10                  15
   His Val Ala Arg Ala Asn Val Lys His Leu Lys Ile Leu Asn Thr Pro
               20                  25                  30
   Asn Cys Ala Leu Gln Ile Val Ala Arg Leu Lys Asn Asn Asn Arg Gln
           35                  40                  45
   Val Cys Ile Asp Pro Lys Leu Lys Trp Ile Gln Glu Tyr Leu Glu Lys
       50                  55                  60
   Ala Leu Asn
   65
<210> 11
   <211> 66
   <212> PRT
   <213> Artificial sequence
<220>
   <223> Synthetic peptide
<220>
   <221> X
   <222> (64)..(64)
   <223> X is FmocLys(ivDde)
<400> 11
   Lys Pro Val Ser Leu Ser Tyr Arg Cys Pro Cys Arg Phe Phe Glu Ser
   1               5                   10                  15
   His Val Ala Arg Ala Asn Val Lys His Leu Lys Ile Leu Asn Thr Pro
               20                  25                  30
   Asn Cys Ala Leu Gln Ile Val Ala Arg Leu Lys Asn Asn Asn Arg Gln
           35                  40                  45
   Val Cys Ile Asp Pro Lys Leu Lys Trp Ile Gln Glu Tyr Leu Glu Ala
       50                  55                  60
   Leu Asn
   65
<210> 12
   <211> 67
   <212> PRT
   <213> Artificial sequence
<220>
   <223> Synthetic peptide
<220>
   <221> X
   <222> (64)..(64)
   <223> X is an amino acid residue that can be biotinylated, such as lysine, ornithine or diaminopropionic acid and optionally is biotinylated, optionally via a spacer molecule such as PEG, e.g. K(PEG-Biotin)
<400> 12
   Lys Pro Val Ser Leu Ser Tyr Arg Cys Pro Cys Arg Phe Phe Glu Ser
   1               5                   10                  15
   His Val Ala Arg Ala Asn Val Lys His Leu Lys Ile Leu Asn Thr Pro
               20                  25                  30
   Asn Cys Ala Leu Gln Ile Val Ala Arg Leu Lys Asn Asn Asn Arg Gln
           35                  40                  45
   Val Cys Ile Asp Pro Lys Leu Lys Trp Ile Gln Glu Tyr Leu Glu Xaa
       50                  55                  60
   Ala Leu Asn
   65
<210> 13
   <211> 69
   <212> PRT
   <213> Artificial sequence
<220>
   <223> Synthetic peptide
<400> 13
   Gly Pro Tyr Gly Ala Asn Met Glu Asp Ser Val Cys Cys Arg Asp Tyr
   1               5                   10                  15
   Val Arg Tyr Arg Leu Pro Leu Arg Val Val Lys His Phe Tyr Trp Thr
               20                  25                  30
   Ser Asp Ser Cys Pro Arg Pro Gly Val Val Leu Leu Thr Phe Arg Asp
           35                  40                  45
   Lys Glu Ile Cys Ala Asp Pro Arg Val Pro Trp Val Lys Met Ile Leu
       50                  55                  60
   Asn Lys Leu Ser Gln
   65
<210> 14
   <211> 69
   <212> PRT
   <213> Artificial sequence
<220>
   <223> Synthetic peptide
<220>
   <221> X
   <222> (66)..(66)
   <223> X is FmocLys(ivDde)
<400> 14
   Gly Pro Tyr Gly Ala Asn Met Glu Asp Ser Val Cys Cys Arg Asp Tyr
   1               5                   10                  15
   Val Arg Tyr Arg Leu Pro Leu Arg Val Val Lys His Phe Tyr Trp Thr
               20                  25                  30
   Ser Asp Ser Cys Pro Arg Pro Gly Val Val Leu Leu Thr Phe Arg Asp
           35                  40                  45
   Lys Glu Ile Cys Ala Asp Pro Arg Val Pro Trp Val Lys Met Ile Leu
       50                  55                  60
   Asn Xaa Leu Ser Gln
   65
<210> 15
   <211> 69
   <212> PRT
   <213> Artificial sequence
<220>
   <223> Synthetic peptide
<220>
   <221> X
   <222> (66)..(66)
   <223> X is an amino acid residue that can be biotinylated, such as lysine, ornithine or diaminopropionic acid and optionally is biotinylated, optionally via a spacer molecule such as PEG, especially K(PEG-Biotin)
<400> 15
   Gly Pro Tyr Gly Ala Asn Met Glu Asp Ser Val Cys Cys Arg Asp Tyr
   1               5                   10                  15
   Val Arg Tyr Arg Leu Pro Leu Arg Val Val Lys His Phe Tyr Trp Thr
               20                  25                  30
   Ser Asp Ser Cys Pro Arg Pro Gly Val Val Leu Leu Thr Phe Arg Asp
           35                  40                  45
   Lys Glu Ile Cys Ala Asp Pro Arg Val Pro Trp Val Lys Met Ile Leu
       50                  55                  60
   Asn Xaa Leu Ser Gln
   65
<210> 16
   <211> 72
   <212> PRT
   <213> Artificial sequence
<220>
   <223> Synthetic peptide
<220>
   <221> X
   <222> (72)..(72)
   <223> X is an amino acid residue that can be biotinylated, such as lysine, ornithine or diaminopropionic acid and optionally is biotinylated (e.g. K-biotin), optionally via a spacer molecule such as PEG, in particular K(PEG-Biotin)
<400> 16
   Ala Arg Gly Thr Asn Val Gly Arg Glu Cys Cys Leu Glu Tyr Phe Lys
   1               5                   10                  15
   Gly Ala Ile Pro Leu Arg Lys Leu Lys Thr Trp Tyr Gln Thr Ser Glu
               20                  25                  30
   Asp Cys Ser Arg Asp Ala Ile Val Phe Val Thr Val Gln Gly Arg Ala
           35                  40                  45
   Ile Cys Ser Asp Pro Asn Asn Lys Arg Val Lys Asn Ala Val Lys Tyr
       50                  55                  60
   Leu Gln Ser Leu Glu Arg Ser Xaa
   65                  70
<210> 17
   <211> 72
   <212> PRT
   <213> Artificial sequence
<220>
   <223> Synthetic peptide
<220>
   <221> X
   <222> (72)..(72)
   <223> X is FmocLys(ivDde)
<400> 17
   Ala Arg Gly Thr Asn Val Gly Arg Glu Cys Cys Leu Glu Tyr Phe Lys
   1 5 10 15
   Gly Ala Ile Pro Leu Arg Lys Leu Lys Thr Trp Tyr Gln Thr Ser Glu
   20 25 30
   Asp Cys Ser Arg Asp Ala Ile Val Phe Val Thr Val Gln Gly Arg Ala
   35 40 45
   Ile Cys Ser Asp Pro Asn Asn Lys Arg Val Lys Asn Ala Val Lys Tyr
   50 55 60
   Leu Gln Ser Leu Glu Arg Ser Xaa
   65 70
<210> 18
   <211> 72
   <212> PRT
   <213> Artificial sequence
<220>
   <223> Synthetic peptide
<220>
   <221> X
   <222> (72)..(72)
   <223> X is K(PEG-Biotin)
<400> 18
   Ala Arg Gly Thr Asn Val Gly Arg Glu Cys Cys Leu Glu Tyr Phe Lys
   1 5 10 15
   Gly Ala Ile Pro Leu Arg Lys Leu Lys Thr Trp Tyr Gln Thr Ser Glu
   20 25 30
   Asp Cys Ser Arg Asp Ala Ile Val Phe Val Thr Val Gln Gly Arg Ala
   35 40 45
   Ile Cys Ser Asp Pro Asn Asn Lys Arg Val Lys Asn Ala Val Lys Tyr
   50 55 60
   Leu Gln Ser Leu Glu Arg Ser Xaa
   65 70
<210> 19
   <211> 78
   <212> PRT
   <213> Artificial sequence
<220>
   <223> Synthetic peptide
<220>
   <221> X
   <222> (78)..(78)
   <223> X is an amino acid residue that can be biotinylated, such as lysine, ornithine or diaminopropionic acid and optionally is biotinylated (e.g. K-biotin), optionally via a spacer molecule such as PEG, in particular K(PEG-Biotin)
<400> 19
   Gly Thr Asn Asp Ala Glu Asp Cys Cys Leu Ser Val Thr Gln Lys Pro
   1               5                   10                  15
   Ile Pro Gly Tyr Ile Val Arg Asn Phe His Tyr Leu Leu Ile Lys Asp
               20                  25                  30
   Gly Cys Arg Val Pro Ala Val Val Phe Thr Thr Leu Arg Gly Arg Gln
           35                  40                  45
   Leu Cys Ala Pro Pro Asp Gln Pro Trp Val Glu Arg Ile Ile Gln Arg
       50                  55                  60
   Leu Gln Arg Thr Ser Ala Lys Met Lys Arg Arg Ser Ser Xaa
   65                  70                  75
<210> 20
   <211> 78
   <212> PRT
   <213> Artificial sequence
<220>
   <223> Synthetic peptide
<220>
   <221> X
   <222> (78)..(78)
   <223> X is FmocLys(ivDde)
<400> 20
   Gly Thr Asn Asp Ala Glu Asp Cys Cys Leu Ser Val Thr Gln Lys Pro
   1               5                   10                  15
   Ile Pro Gly Tyr Ile Val Arg Asn Phe His Tyr Leu Leu Ile Lys Asp
               20                  25                  30
   Gly Cys Arg Val Pro Ala Val Val Phe Thr Thr Leu Arg Gly Arg Gln
           35                  40                  45
   Leu Cys Ala Pro Pro Asp Gln Pro Trp Val Glu Arg Ile Ile Gln Arg
       50                  55                  60
   Leu Gln Arg Thr Ser Ala Lys Met Lys Arg Arg Ser Ser Xaa
   65                  70                  75
<210> 21
   <211> 78
   <212> PRT
   <213> Artificial sequence
<220>
   <223> Synthetic peptide
<220>
   <221> X
   <222> (78)..(78)
   <223> X is biotinylated lysine
<400> 21
   Gly Thr Asn Asp Ala Glu Asp Cys Cys Leu Ser Val Thr Gln Lys Pro
   1               5                   10                  15
   Ile Pro Gly Tyr Ile Val Arg Asn Phe His Tyr Leu Leu Ile Lys Asp
               20                  25                  30
   Gly Cys Arg Val Pro Ala Val Val Phe Thr Thr Leu Arg Gly Arg Gln
           35                  40                  45
   Leu Cys Ala Pro Pro Asp Gln Pro Trp Val Glu Arg Ile Ile Gln Arg
       50                  55                  60
   Leu Gln Arg Thr Ser Ala Lys Met Lys Arg Arg Ser Ser Xaa
   65                  70                  75
<210> 22
   <211> 73
   <212> PRT
   <213> Artificial sequence
<220>
   <223> Synthetic peptide
<400> 22
   Phe Pro Met Phe Lys Arg Gly Arg Cys Leu Cys Ile Gly Pro Gly Val
   1               5                   10                  15
   Lys Ala Val Lys Val Ala Asp Ile Glu Lys Ala Ser Ile Met Tyr Pro
               20                  25                  30
   Ser Asn Asn Cys Asp Lys Ile Glu Val Ile Ile Thr Leu Lys Glu Asn
           35                  40                  45
   Lys Gly Gln Arg Cys Leu Asn Pro Lys Ser Lys Gln Ala Arg Leu Ile
       50                  55                  60
   Ile Lys Lys Val Glu Arg Lys Asn Phe
   65                  70
<210> 23
   <211> 74
   <212> PRT
   <213> Artificial sequence
<220>
   <223> Synthetic peptide
<220>
   <221> X
   <222> (74)..(74)
   <223> X =a residue that can be biotinylated, such as lysine, ornithine or diaminopropionic acid and optionally is biotinylated, optionally via a spacer molecule such as PEG. The amino acid residue may be added via a spacer molecule such as PEG.
<400> 23
   Phe Pro Met Phe Lys Arg Gly Arg Cys Leu Cys Ile Gly Pro Gly Val
   1               5                   10                  15
   Lys Ala Val Lys Val Ala Asp Ile Glu Lys Ala Ser Ile Met Tyr Pro
               20                  25                  30
   Ser Asn Asn Cys Asp Lys Ile Glu Val Ile Ile Thr Leu Lys Glu Asn
           35                  40                  45
   Lys Gly Gln Arg Cys Leu Asn Pro Lys Ser Lys Gln Ala Arg Leu Ile
       50                  55                  60
   Ile Lys Lys Val Glu Arg Lys Asn Phe Xaa
   65                  70
<210> 24
   <211> 74
   <212> PRT
   <213> Artificial sequence
<220>
   <223> Synthetic peptide
<220>
   <221> X
   <222> (74)..(74)
   <223> X is PEG-K(ivDde)
<400> 24
   Phe Pro Met Phe Lys Arg Gly Arg Cys Leu Cys Ile Gly Pro Gly Val
   1               5                   10                  15
   Lys Ala Val Lys Val Ala Asp Ile Glu Lys Ala Ser Ile Met Tyr Pro
               20                  25                  30
   Ser Asn Asn Cys Asp Lys Ile Glu Val Ile Ile Thr Leu Lys Glu Asn
           35                  40                  45
   Lys Gly Gln Arg Cys Leu Asn Pro Lys Ser Lys Gln Ala Arg Leu Ile
       50                  55                  60
   Ile Lys Lys Val Glu Arg Lys Asn Phe Xaa
   65                  70
<210> 25
   <211> 74
   <212> PRT
   <213> Artificial sequence
<220>
   <223> Synthesised peptide
<220>
   <221> X
   <222> (74)..(74)
   <223> X is PEG-K(Biotin)
<400> 25
   Phe Pro Met Phe Lys Arg Gly Arg Cys Leu Cys Ile Gly Pro Gly Val
   1               5                   10                  15
   Lys Ala Val Lys Val Ala Asp Ile Glu Lys Ala Ser Ile Met Tyr Pro
               20                  25                  30
   Ser Asn Asn Cys Asp Lys Ile Glu Val Ile Ile Thr Leu Lys Glu Asn
           35                  40                  45
   Lys Gly Gln Arg Cys Leu Asn Pro Lys Ser Lys Gln Ala Arg Leu Ile
       50                  55                  60
   Ile Lys Lys Val Glu Arg Lys Asn Phe Xaa
   65                  70
<210> 26
   <211> 74
   <212> PRT
   <213> Artificial sequence
<220>
   <223> Synthesised peptide
<220>
   <221> X
   <222> (1)..(1)
   <223> X = pyroGlu or Gln
<220>
   <221> X
   <222> (64)..(64)
   <223> X = Norleucine
<220>
   <221> X
   <222> (72)..(72)
   <223> X is an amino acid residue that can be biotinylated, such as lysine, ornithine or diaminopropionic acid and optionally is biotinylated, optionally via a spacer molecule such as PEG, e.g. K(PEG-Biotin)
<400> 26
   Xaa Gly Val Phe Glu Asp Cys Cys Leu Ala Tyr His Tyr Pro Ile Gly
   1               5                   10                  15
   Trp Ala Val Leu Arg Arg Ala Trp Thr Tyr Arg Ile Gln Glu Val Ser
               20                  25                  30
   Gly Ser Cys Asn Leu Pro Ala Ala Ile Phe Tyr Leu Pro Lys Arg His
           35                  40                  45
   Arg Lys Val Cys Gly Asn Pro Lys Ser Arg Glu Val Gln Arg Ala Xaa
       50                  55                  60
   Lys Leu Leu Asp Ala Arg Asn Xaa Val Phe
   65                  70
<210> 27
   <211> 74
   <212> PRT
   <213> Artificial sequence
<220>
   <223> Synthesised peptide
<220>
   <221> X
   <222> (1)..(1)
   <223> X = pyroGlu or Gln
<220>
   <221> X
   <222> (64)..(64)
   <223> X = Norleucine
<220>
   <221> X
   <222> (72)..(72)
   <223> X is K(ivDde)
<400> 27
   Xaa Gly Val Phe Glu Asp Cys Cys Leu Ala Tyr His Tyr Pro Ile Gly
   1               5                   10                  15
   Trp Ala Val Leu Arg Arg Ala Trp Thr Tyr Arg Ile Gln Glu Val Ser
               20                  25                  30
   Gly Ser Cys Asn Leu Pro Ala Ala Ile Phe Tyr Leu Pro Lys Arg His
           35                  40                  45
   Arg Lys Val Cys Gly Asn Pro Lys Ser Arg Glu Val Gln Arg Ala Xaa
       50                  55                  60
   Lys Leu Leu Asp Ala Arg Asn Xaa Val Phe
   65                  70
<210> 28
   <211> 74
   <212> PRT
   <213> Artificial sequence
<220>
   <223> Synthesised peptide
<220>
   <221> X
   <222> (1)..(1)
   <223> X = pyroGlu or Gln
<220>
   <221> X
   <222> (64)..(64)
   <223> X = Norleucine
<220>
   <221> X
   <222> (72)..(72)
   <223> X is K(PEG-Biotin)
<400> 28
   Xaa Gly Val Phe Glu Asp Cys Cys Leu Ala Tyr His Tyr Pro Ile Gly
   1               5                   10                  15
   Trp Ala Val Leu Arg Arg Ala Trp Thr Tyr Arg Ile Gln Glu Val Ser
               20                  25                  30
   Gly Ser Cys Asn Leu Pro Ala Ala Ile Phe Tyr Leu Pro Lys Arg His
           35                  40                  45
   Arg Lys Val Cys Gly Asn Pro Lys Ser Arg Glu Val Gln Arg Ala Xaa
       50                  55                  60
   Lys Leu Leu Asp Ala Arg Asn Xaa Val Phe
   65                  70
<210> 29
   <211> 88
   <212> PRT
   <213> Artificial sequence
<220>
   <223> Synthesised peptide
<220>
   <221> X
   <222> (87)..(87)
   <223> X is an amino acid residue that can be biotinylated, such as lysine, ornithine or diaminopropionic acid and optionally is biotinylated, optionally via a spacer molecule such as PEG, e.g. K(PEG-Biotin)
<400> 29
   Phe Leu Leu Pro Pro Ser Thr Ala Cys Cys Thr Gln Leu Tyr Arg Lys
   1               5                   10                  15
   Pro Leu Ser Asp Lys Leu Leu Arg Lys Val Ile Gln Val Glu Leu Gln
               20                  25                  30
   Glu Ala Asp Gly Asp Cys His Leu Gln Ala Phe Val Leu His Leu Ala
           35                  40                  45
   Gln Arg Ser Ile Cys Ile His Pro Gln Asn Pro Ser Leu Ser Gln Trp
       50                  55                  60
   Phe Glu His Gln Glu Arg Lys Leu His Gly Thr Leu Pro Lys Leu Asn
   65                  70                  75                  80
   Phe Gly Met Leu Arg Lys Xaa Gly
                   85
<210> 30
   <211> 88
   <212> PRT
   <213> Artificial sequence
<220>
   <223> Synthesised peptide
<220>
   <221> X
   <222> (87)..(87)
   <223> X is K(ivDde)
<400> 30
   Phe Leu Leu Pro Pro Ser Thr Ala Cys Cys Thr Gln Leu Tyr Arg Lys
   1               5                   10                  15
   Pro Leu Ser Asp Lys Leu Leu Arg Lys Val Ile Gln Val Glu Leu Gln
               20                  25                  30
   Glu Ala Asp Gly Asp Cys His Leu Gln Ala Phe Val Leu His Leu Ala
           35                  40                  45
   Gln Arg Ser Ile Cys Ile His Pro Gln Asn Pro Ser Leu Ser Gln Trp
       50                  55                  60
   Phe Glu His Gln Glu Arg Lys Leu His Gly Thr Leu Pro Lys Leu Asn
   65                  70                  75                  80
   Phe Gly Met Leu Arg Lys Xaa Gly
                   85
<210> 31
   <211> 88
   <212> PRT
   <213> Artificial sequence
<220>
   <223> Synthesised peptide
<220>
   <221> X
   <222> (87)..(87)
   <223> X is K(PEG-Biotin)
<400> 31
   Phe Leu Leu Pro Pro Ser Thr Ala Cys Cys Thr Gln Leu Tyr Arg Lys
   1               5                   10                  15
   Pro Leu Ser Asp Lys Leu Leu Arg Lys Val Ile Gln Val Glu Leu Gln
               20                  25                  30
   Glu Ala Asp Gly Asp Cys His Leu Gln Ala Phe Val Leu His Leu Ala
           35                  40                  45
   Gln Arg Ser Ile Cys Ile His Pro Gln Asn Pro Ser Leu Ser Gln Trp
       50                  55                  60
   Phe Glu His Gln Glu Arg Lys Leu His Gly Thr Leu Pro Lys Leu Asn
   65                  70                  75                  80
   Phe Gly Met Leu Arg Lys Xaa Gly
                   85
<210> 32
   <211> 77
   <212> PRT
   <213> Artificial sequence
<220>
   <223> Synthesised peptide
<400> 32
   Val Pro Leu Ser Arg Thr Val Arg Cys Thr Cys Ile Ser Ile Ser Asn
   1               5                   10                  15
   Gln Pro Val Asn Pro Arg Ser Leu Glu Lys Leu Glu Ile Ile Pro Ala
               20                  25                  30
   Ser Gln Phe Cys Pro Arg Val Glu Ile Ile Ala Thr Met Lys Lys Lys
           35                  40                  45
   Gly Glu Lys Arg Cys Leu Asn Pro Glu Ser Lys Ala Ile Lys Asn Leu
       50                  55                  60
   Leu Lys Ala Val Ser Lys Glu Arg Ser Lys Arg Ser Pro
   65                  70                  75
<210> 33
   <211> 78
   <212> PRT
   <213> Artificial sequence
<220>
   <223> Synthesised peptide
<220>
   <221> X
   <222> (78)..(78)
   <223> X is K(ivDde), optionally attached via a spacer such as PEG, e.g. -PEG-K(ivDde)
<400> 33
   Val Pro Leu Ser Arg Thr Val Arg Cys Thr Cys Ile Ser Ile Ser Asn
   1               5                   10                  15
   Gln Pro Val Asn Pro Arg Ser Leu Glu Lys Leu Glu Ile Ile Pro Ala
               20                  25                  30
   Ser Gln Phe Cys Pro Arg Val Glu Ile Ile Ala Thr Met Lys Lys Lys
           35                  40                  45
   Gly Glu Lys Arg Cys Leu Asn Pro Glu Ser Lys Ala Ile Lys Asn Leu
       50                  55                  60
   Leu Lys Ala Val Ser Lys Glu Arg Ser Lys Arg Ser Pro Xaa
   65                  70                  75
<210> 34
   <211> 78
   <212> PRT
   <213> Artificial sequence
<220>
   <223> Synthesised peptide
<220>
   <221> X
   <222> (78)..(78)
   <223> X =a residue that can be biotinylated, such as lysine, ornithine or diaminopropionic acid and optionally is biotinylated, optionally via a spacer molecule such as PEG. The amino acid residue may be added via a spacer molecule such as PEG.
<400> 34
   Val Pro Leu Ser Arg Thr Val Arg Cys Thr Cys Ile Ser Ile Ser Asn
   1               5                   10                  15
   Gln Pro Val Asn Pro Arg Ser Leu Glu Lys Leu Glu Ile Ile Pro Ala
               20                  25                  30
   Ser Gln Phe Cys Pro Arg Val Glu Ile Ile Ala Thr Met Lys Lys Lys
           35                  40                  45
   Gly Glu Lys Arg Cys Leu Asn Pro Glu Ser Lys Ala Ile Lys Asn Leu
       50                  55                  60
   Leu Lys Ala Val Ser Lys Glu Arg Ser Lys Arg Ser Pro Xaa
   65                  70                  75

## Description

### FIELD OF THE INVENTION

The various embodiments of the present invention relate to binding reagents capable of specifically binding to the chemokine receptor, CCR7, for use in the treatment of cancer, and binding reagents capable of specifically binding to one or more regulatory T cell receptors .

### BACKGROUND OF THE INVENTION

Cancer is a disease characterised by uncontrolled cell growth caused by accumulation of genetic mutations in the cellular DNA. There are over 200 types of different cancer classified according to the cell of origin from which the cancer or tumour first developed. Furthermore, cancers can be classified broadly as either solid tumours, for example breast cancer, colorectal cancer and melanoma, or as haematological malignancies such as leukaemias and lymphomas.

Solid tumours typically initiate and grow as an abnormal mass at a local site. However, during the course of cancer progression, the cells may acquire the ability to invade the underlying tissue and thereby enter the circulatory and/or lymphatic systems. These invasive properties ultimately allow solid tumours to metastasise to distal sites within the body, and it is metastasis combined with growth at secondary sites that accounts for the vast majority of cancer deaths.

In contrast to solid tumours, cancers such as leukaemias and lymphomas derive from cells of haematological origin, and therefore manifest as systemic diseases. In particular, chronic lymphocytic leukaemia, the most common form of leukaemia, develops from malignant lymphocytes originating in the bone marrow.

The body has many intrinsic mechanisms intended to guard against cancer development. In this regard, the immune system is thought to play a key role in eradicating cells harbouring genetic mutations. It follows therefore, that cancer cells often persist by evolving ways to avoid recognition by the cells of the immune system. In particular, it has been shown that elevated levels of T regulatory cells, both within the peripheral circulation and within the tumour microenvironment, underlie the immune suppression seen in cancer patients. The presence of increased numbers of regulatory T cells has also been identified as a barrier to the successful implementation of cancer immunotherapies.

Apheresis is a treatment used for depletion of blood components, such as antibodies, low-density lipoproteins (LDL) and blood cells. Leukapheresis is the apheresis treatment used for removal of white blood cells, leukocytes. The patient is connected to an extracorporeal blood circulating system; the blood is drawn from a vein in one arm, passed through a column device and returned into the other arm of the patient. WO2010/029317 describes apheresis columns useful for treating inflammatory conditions including a chemokine immobilised on a solid support.

WO 2010/029317 relates to a column loaded with a chemokine for use in treatment of inflammatory diseases, such as Crohn's disease.

EP 2 067 495 relates to an adsorption column which may be used for depleting blood from a patient in the treatment of cancer.

### SUMMARY OF THE INVENTION

Chemokines are a class of cytokine molecules involved in cell recruitment and activation in inflammation. Chemokines cause chemotaxis and activation of various subpopulations of cells in the immune system. The activity of chemokines is mediated primarily through tight binding to their receptors on the surface of leukocytes. In certain embodiments the present invention is based on the realisation that the interaction between chemokines and cells expressing their receptors may be exploited for the treatment of various cancers and components thereof, such as by reducing levels of circulating tumour cells, reducing the incidence of tumour metastasis, removing regulatory T lymphocytes (which may be referred to herein as "Tregs") from the peripheral blood, or treating leukaemias such as chronic lymphocytic leukaemia, chronic myeloid leukemia, use for debulking in AML, ALL and before harvest for autologous bone marrow /stem cell transplantation and for treating the leukemic phase of lymphoma. The inventors have determined that targeting specific chemokine receptor-expressing cells presents a new therapeutic approach to treat such conditions. Moreover, in such conditions, chemokine receptor expression on each cell may be increased again providing a therapeutic approach to treat such conditions. It is shown herein that cancer patients, in particular subjects suffering from UBC and PC, exhibit an increased frequency of CCR4 expressing circulating Tregs and that this response is specific to Tregs (and does not apply to other T lymphocytes). It is shown herein that CCR7 expressing B cells may be efficiently depleted using MIP3b as a specific binding reagent in a leukapheresis method.

Thus, in certain embodiments the invention serves to reduce the levels of circulating tumour cells, reduce the incidence of tumour metastasis, remove regulatory T lymphocytes from the peripheral blood or treat leukaemias such as chronic lymphocytic leukaemia, chronic myeloid leukemia. In certain embodiments the invention may also be used for debulking in AML, ALL and before harvest for autologous bone marrow /stem cell transplantation and to treat the leukemic phase of lymphoma. This is achieved using specific binding reagents to capture specific chemokine receptor-expressing cancer cells and regulatory T lymphocytes from the patient. Accordingly, in certain embodiments the invention provides in a first aspect a binding reagent capable of specifically binding to the chemokine receptor, CCR7, for use in the treatment of cancer, wherein the binding reagent is immobilized on a solid support contained within an apheresis column, to which is applied peripheral blood from a patient thus removing CCR7 expressing cells from the peripheral blood of the patient, wherein the binding reagent is a chemokine is selected from CCL19 or CCL21.

In certain embodiments the invention therefore also provides a binding reagent capable of specifically binding to one or more regulatory T cell receptors, in particular the cutaneous lymphocyte antigen (CLA) receptor, for use in a method for treating cancer wherein the binding reagent is immobilized directly or indirectly on a solid support contained within an apheresis column, to which is applied peripheral blood from a patient thus removing one or more regulatory T cell receptor-expressing cells (Tregs) from the peripheral blood of the patient and wherein the binding reagent is capable of binding to CCR7 wherein the binding reagent is a chemokine is selected from CCL19 or CCL21.

The expression of chemokine receptors may vary from leukaemia to leukaemia. For example, the difference between a leukaemia with circulating cells and lymphoma with lymph node malignant cell may lie in the expression of CCR7 allowing entrance into the lymph node. When lymphoma enters into a leukemic phase it is likely due to down-regulation of CCR7. Accordingly, in other embodiments the invention also enables a method for treating leukemia comprising applying peripheral blood from a patient to an apheresis column loaded with a solid support comprising one or more binding reagents capable of specifically binding to one or more chemokine receptors, which have been identified as being linked to leukemia, for example up-regulated in leukemia, immobilized directly or indirectly on the support thus removing one or more chemokine receptor expressing cells from the peripheral blood of the patient. Suitable chemokine receptor expressing cells can be identified by applying the methods described herein.

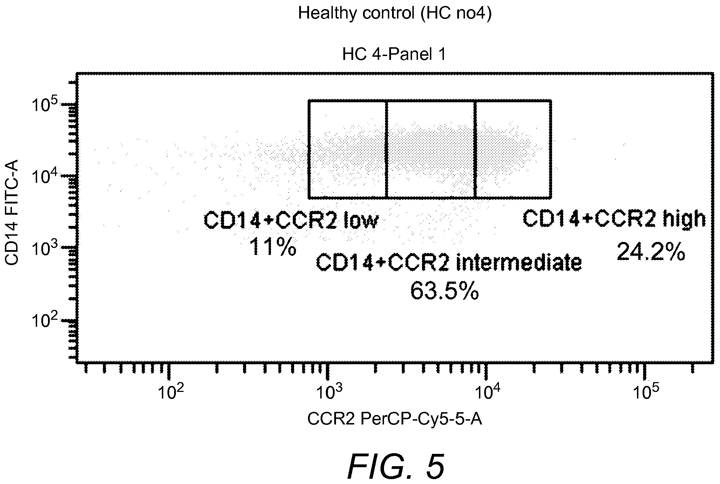

As shown herein, suitable binding reagents can be immobilized onto a solid support, either directly or indirectly, to generate an apheresis column suitable for capturing relevant chemokine receptor-expressing cells. Where increased levels of chemokine receptor expression are observed, such cells may be preferably removed from the peripheral blood using the columns of the various embodiments of the invention. Thus, the methods of various embodiments of the invention may preferably target CCR7hi cells as defined herein for removal from the peripheral blood. "High" expression may be determined according to standard flow cytometry techniques. The level is measured relative to levels of expression of the chemokine receptor in cells taken from a healthy subject. Figure 5 provides an example of a gating strategy.

In certain embodiments the invention further provides a binding reagent capable of specifically binding to chemokine receptor CCR7 for use in the treatment of cancer, wherein the one or more binding reagents is immobilized, directly or indirectly, on a solid support contained within an apheresis column, to which is applied peripheral blood from a patient thus removing one or more CCR7 expressing cells from the peripheral blood of the patient

In certain embodiments the invention aims to treat a range of specific cancer, or cancer-related, conditions. Specific conditions may be selected from reducing levels of circulating tumour cells, reducing the incidence of tumour metastasis, removing regulatory T lymphocytes from the peripheral blood, or treating leukaemias such as chronic lymphocytic leukaemia, chronic myeloid leukemia, use for debulking in AML, ALL and before harvest for autologous bone marrow /stem cell transplantation and treating the leukemic phase of lymphoma. Reducing levels of circulating tumour cells may be applicable to a range of cancers. This aspect may be relevant to certain solid tumours such as breast cancer, colon cancer or lung cancer. Reducing the incidence of tumour metastasis may rely upon preventing metastatic cell entry at specific locations, since this may be driven via chemokine-chemokine receptor interactions. Thus, in certain embodiments the methods of the invention may be utilized to prevent entry of metastatic cells into any one or more of bone, lungs, lymph nodes, skin and small intestine. In one embodiment, CCR7 expressing cells are targeted to prevent metastatic cell entry into the lymph nodes.

Removal of regulatory T lymphocytes (which may be referred to herein as "Tregs") from the peripheral blood may assist with immune system recognition of the cancer or tumour. T regulatory cells (Tregs) are a subpopulation of T cells that suppress the immune system in order to maintain immune homeostasis. In cancer, the Tregs can inhibit an effective immune response against the tumor and thus removal of the Tregs may lead to an improved immune activation against the cancer cells. In certain embodiments this aspect of the invention may thus be applicable to treating any cancer. Specific examples include pancreatic cancer and bladder cancer, in particular urinirary bladder cancer.

In certain embodiments the methods of the invention may also be utilized in combination with other forms of therapy, such as chemotherapies. Debulking using the leukaphereis procedure may decrease the need for chemotherapy and therefore diminish side effects.

Treating leukaemias such as chronic lymphocytic leukaemia and chronic myeloid leukemia may be based upon direct removal of cancerous cells in the peripheral blood. Use for debulking in AML and ALL may rely upon the targeted cytoreductive effects achieved by the various embodiments of the present invention. Similarly, removal of cancerous cells or cells contributing to the cancerous condition (such as Tregs) may be useful prior to harvest of bone marrow or stem cells from a subject or patient for autologous bone marrow /stem cell transplantation. In certain embodiments the invention may also be useful for treating the leukemic phase of lymphoma, when excess lymphocytes are found in the peripheral blood. The excess lymphocytes, which may include Tregs, can be removed on the basis of the cells expressing particular receptors, especially chemokine receptors.

The three major types of lymphocyte are T cells, B cells and natural killer (NK) cells. The term "T-lymphocyte" includes CD4⁺ T cells such as T helper cells (Th1 cells and Th2 cells), and CD8⁺ T cells such as cytotoxic T cells. Th1 cells may be characterized by expression of CCR5 and/or by production of IFN-γ. Th2 cells may be characterized by expression of CCR3 and/or by production of IL-4. Regulatory T cells (Tregs) express the transcription factor FoxP3 and express high levels of the IL-2Ra (CD25) on the cell surface. In addition they have down-regulated levels of the IL-7a receptor CD127. Thus, Tregs can be defined as CD4+CD25hiCD127lo/- and Foxp3 positive. The function of Tregs is to regulate and modulate T effector cell responses by direct and indirect contacts mediated by cytokines. Thus, tumors induce Tregs in order to avoid recognition and elimination by T effector cells as a tumor escape mechanism.

In certain embodiments the methods of the invention may involve specific binding interactions with any one or more of these further cell-surface (and cell-specific) markers in addition to the removal based upon binding to chemokine receptor CCR7. Suitable binding reagents can be prepared to specifically bind to these cell-surface markers.

CCR5 is the gene symbol approved by the HUGO Gene Nomenclature Committee for chemokine (C-C motif) receptor 5. The HGNC ID for this gene is 1605. The gene is located at chromosome position 3p21. The previous symbol and name for the gene is CMKBR5. Synonyms for this gene include CC-CKR-5, CD195 CKR-5, IDDM22 and CKR5. The Entrez Gene reference sequence for CCR5 is 1234 as available on 13 June 2011.

CCR6 is the gene symbol approved by the HUGO Gene Nomenclature Committee for chemokine (C-C motif) receptor 6. The HGNC ID for this gene is 1607. The gene is located at chromosome position 6q27. The previous symbol and name for the gene is STRL22. Synonyms for this gene include BN-1, CD196, CKR-L3, CMKBR6, DCR2, DRY-6, GPR-CY4, GPR29. The Genbank reference sequence for CCR6 is U68030.1 as available on 13 June 2011.

CCR7 is the gene symbol approved by the HUGO Gene Nomenclature Committee for chemokine (C-C motif) receptor 7. The HGNC ID for this gene is 1608. The gene is located at chromosome position 17q12-q21.2. The previous symbol and name for the gene is CMKBR7, EBI1. Synonyms for this gene include BLR2, CD197 and CDw197. The RefSeq reference sequence for CCR1 is NM_001838.3 as available on 13 June 2011.

CCR8 is the gene symbol approved by the HUGO Gene Nomenclature Committee for chemokine (C-C motif) receptor 8. The HGNC ID for this gene is 1609. The gene is located at chromosome position 3p22. The previous symbol and name for the gene is CMKBR8, CMK. Synonyms for this gene include CDw198, CKR-L1, CY6, GPR-CY6, TER1. The Genbank reference sequence for CCR8 is D49919.1 as available on 13 June 2011.

CXCR4 is the gene symbol approved by the HUGO Gene Nomenclature Committee for chemokine (C-X-C motif) receptor 4. The HGNC ID for this gene is 2561. The gene is located at chromosome position 2q21. The previous symbol and name for the gene is "chemokine (C-X-C motif), receptor 4 (fusin)". Synonyms for this gene include CD184, D2S201E, fusin, HM89, HSY3RR, LESTR, NPY3R, NPYR, NPYY3R. The Genbank reference sequence for CXCR4 is AJ132337.1 as available on 13 June 2011.

CXCR7 is the gene symbol approved by the HUGO Gene Nomenclature Committee for chemokine (C-X-C motif) receptor 7. The HGNC ID for this gene is 23692. The gene is located at chromosome position 2q37.3. The previous symbol and name for the gene is "chemokine orphan receptor 1", CMKOR1. Synonyms for this gene include GPR159 and RDC1. The Genbank reference sequence for CXCR7 is BC008459.1 as available on 13 June 2011.

CCR4 is the gene symbol approved by the HUGO Gene Nomenclature Committee for chemokine (C-C motif) receptor 4. The HGNC ID for this gene is 1605. The gene is located at chromosome position 3p24-p21.3. Synonyms for this gene include CC-CKR-4, CD194, ChemR13, CKR4, CMKBR4, k5-5. The Genbank reference sequence for CCR4 is X85740.1 as available on 4 April 2012.

Cutaneous lymphocyte antigen (CLA herein) is a specialized form of PSGL-1 expressed on skin-homing T cells, see Fuhlbrigge et al., Nature 389, 978-981 (30 October 1997) | doi:10.1038/40166 (incorporated herein by reference in its entirety). Memory T cells that infiltrate the skin express a unique skin-homing receptor called cutaneous lymphocyte-associated antigen (CLA), a carbohydrate epitope that facilitates the targeting of T cells to inflamed skin1, CLA is an inducible carbohydrate modification of P-selectin glycoprotein ligand-1 (PSGL-1), a known surface glycoprotein that is expressed constitutively on all human peripheral-blood T cells.

CXCR3 is the gene symbol approved by the HUGO Gene Nomenclature Committee for chemokine (C-X-C motif) receptor 3. The HGNC ID for this gene is 4540. The gene is located at chromosome position Xq13. The previous symbol and name for the gene is "G protein-coupled receptor 9", GPR9. Synonyms for this gene include CD183, CKR-L2, CMKAR3, IP10-R and MigR. The Genbank reference sequence for CXCR3 is U32674.1 as available on 13 June 2011.

CXCR5 is the gene symbol approved by the HUGO Gene Nomenclature Committee for chemokine (C-X-C motif) receptor 5. The HGNC ID for this gene is 1060. The gene is located at chromosome position 11q23.3. The previous symbol and name for the gene is BLR1, "Burkitt lymphoma receptor 1, GTP binding protein (chemokine (C-X-C motif) receptor 5)", "Burkitt lymphoma receptor 1, GTP-binding protein". Synonyms for this gene include CD185, MDR15. The Genbank reference sequence for CXCR4 is X68829.1 as available on 29 May 2012.

CCR9 is the gene symbol approved by the HUGO Gene Nomenclature Committee for chemokine (C-C motif) receptor 9. The HGNC ID for this gene is 1610. The gene is located at chromosome position 3p22. The previous symbol and name for the gene is GPR28. Synonyms for this gene include CDw199, GPR-9-6. The Genbank reference sequence for CCR9 is AJ132337.1 as available on 13 June 2011.

CCR10 is the gene symbol approved by the HUGO Gene Nomenclature Committee for chemokine (C-C motif) receptor 10. The HGNC ID for this gene is 4474. The gene is located at chromosome position 17p21.1-q21.3. The previous symbol and name for the gene is "G protein-coupled receptor 2", GPR2. The Genbank reference sequence for CCR9 is AF215981.1 as available on 29 May 2012.

In certain embodiments the invention relies upon a binding reagent which is capable of specifically binding to a chemokine receptor, or to a regulatory T cell receptor in some embodiments. This specific binding reaction permits cells expressing the chemokine receptor or regulatory T cell receptor to be removed from the peripheral blood of the patient when the blood is passed over the solid support upon or within which the binding reagent is immobilized. Specific chemokine receptors of interest include CCR7 (when treating cancers such as leukemias). The binding reagent can be any binding reagent capable of specifically binding to the receptor in question. By "specific binding" is meant that the binding reagent displays sufficient specificity of binding and appropriate binding affinity/kinetics to permit removal of cells expressing CCR7. Whilst it is not precluded that the binding reagent is capable of binding to other molecules, such as other chemokine receptors, the binding reagent will preferentially bind to cells expressing CCR7, and in particular to cells expressing increased levels of CCR7. The binding reagent capable of specifically binding to CCR7 may be either an agonist or an antagonist of CCR7. As the disease condition may rely upon up-regulation of expression of, or signaling via CCR7, in certain embodiments the binding reagent capable of specifically binding to CCR7 is an antagonist of CCR7. Chemokines are typically, although not necessarily exclusively (particularly in the case of truncated or modified forms) agonists of their cognate receptor and serve to activate the cells expressing the relevant receptor, as would be appreciated by one skilled in the art.

In the context of the various embodiments of the present invention the term "chemokine" also comprises biotinylated or otherwise labelled chemokines. The term "chemokine" also comprises modified and truncated versions of the chemokine and chemokine fragments with the proviso that the modified or truncated form retains its ability to bind to its cognate receptor (and thus remains functional in the context of the invention). The chemokine does not necessarily need to retain biological activity as it is specific binding affinity CCR7. In certain embodiments, the chemokine lacks biological activity, for example in terms of activation of the CCR7 receptor

Specific chemokines useful in the various embodiments of the present invention for binding to CCR7 include CCL19 and CCL21.

CCL19 is the gene symbol approved by the HUGO Gene Nomenclature Committee for chemokine (C-C motif) ligand 19, also known as MIP-3b. The HGNC ID for this gene is 10617. The gene is located at chromosome position 9p13. The previous symbol and name for the gene is SCYA19, "small inducible cytokine subfamily A (Cys-Cys), member 19". Synonyms for this gene include "beta chemokine exodus-3", "CC chemokine ligand 19", "CK beta-11", CKb11, "EBI1-ligand chemokine", ELC, exodus-3, "macrophage inflammatory protein 3-beta", MIP-3b. The Genbank reference sequence for CCL19 is AB000887.1 as available on 13 June 2011.

CCL21 is the gene symbol approved by the HUGO Gene Nomenclature Committee for chemokine (C-C motif) ligand 21. The HGNC ID for this gene is 10620. The gene is located at chromosome position 9p13. The previous symbol and name for the gene is SCYA21, "small inducible cytokine subfamily A (Cys-Cys), member 21". Synonyms for this gene include 6Ckine, "beta chemokine exodus-2", CKb9, ECL, "Efficient Chemoattractant for Lymphocytes", exodus-2, "secondary lymphoid tissue chemokine", SLC, TCA4. The Genbank reference sequence for CCL21 is AB002409.1 as available on 13 June 2011.

An example of a chemokine containing modifications is described in detail herein (see Reference Example 3 below). The modified CCL8 (MCP-2) corresponds to residues 1 to 76 of the full length mature protein (and lacks the N-terminal signal peptide of 23 amino acids, which is cleaved off) and thus retains the chemokine fold. The Gln at the N-terminus of the protein is subject to pyroGlu formation under physiological conditions. Thus Gln1 of the sequence is substituted with pyroglutamine to prevent mixed species of N-terminal Gln and pyroGlu being generated (SEQ ID NO: 1). This improves the yield of synthesis and ensures a homogeneous chemokine preparation through column manufacture and use. FmocLys(ivDde)-OH is incorporated as residue 75 to facilitate site-specific labelling at this position of the protein (SEQ ID NO: 2). The naturally occurring lysine at position 75 is modified through biotinylation. A PEG spacer may be incorporated between the ε-amino functionality and the biotin (SEQ ID NO: 3).

Thus, exemplified herein is a modified chemokine comprising, consisting essentially of or consisting of the amino acid sequence of SEQ ID NO: 1:
X1 = pyroGlu (but may remain as Gln in some embodiments)
X75 = an amino acid residue that can be biotinylated, such as lysine, ornithine or diaminopropionic acid and optionally is biotinylated, optionally via a spacer molecule such as PEG, e.g. K(PEG-Biotin).

X1 = pyroGlu (but may remain as Gln in some embodiments)
X75 = K(PEG-Biotin).

A further example of a chemokine is described in detail herein (see Reference Example 4 below). The modified CCL5 (RANTES) corresponds to residues 1 to 68 of the full length mature protein (and lacks the N-terminal signal peptide of 23 amino acids, which is cleaved off) and thus retains the chemokine fold. The single methionine (Met67) within the sequence is mutated to lysine, to mitigate against oxidation of this residue during the chain assembly (SEQ ID NO: 4). This Met to Lys substitution provides a lysine at position 67 which can be modified through biotinylation. FmocLys(ivDde)-OH is incorporated as residue 67 to facilitate site-specific labelling at this position of the protein (SEQ ID NO: 5). The biotinylated version comprises, consists essentially of or consists of the amino acid sequence of SEQ ID NO: 6.

Thus, exemplified herein is a modified chemokine comprising, consisting essentially of or consisting of the amino acid sequence of SEQ ID NO: 6: X is an amino acid residue that can be biotinylated, such as lysine, ornithine or diaminopropionic acid and optionally is biotinylated, optionally via a spacer molecule such as PEG (e.g. K(Biotin))

A further example of a chemokine is described in detail herein (see Reference Example 5 below). The modified CCL20 (MIP-3α) corresponds to residues 1 to 70 of the full length mature protein (and lacks the N-terminal signal peptide of 26 amino acids, which is cleaved off) and thus retains the chemokine fold (SEQ ID NO: 7). FmocLys(ivDde)-OH is incorporated as residue 68 to facilitate site-specific labelling at this position of the protein (SEQ ID NO: 8). The naturally occurring lysine at position 68 is modified through biotinylation. A PEG spacer may be incorporated between the ε-amino functionality and the biotin. The final protein may thus comprise, consist essentially of or consist of the amino acid sequence of SEQ ID NO: 9.

Thus, exemplified herein is a modified chemokine comprising, consisting essentially of or consisting of the amino acid sequence of SEQ ID NO: 9: X is an amino acid residue that can be biotinylated, such as lysine, ornithine or diaminopropionic acid and optionally is biotinylated, optionally via a spacer molecule such as PEG, in particular K(PEG-Biotin)

A further example of a chemokine is described in detail herein (see Reference Example 6 below). The truncated CXCL12 (SDF-1α) corresponds to residues 1 to 67 of the full length mature protein (and lacks the N-terminal signal peptide of 21 amino acids, which is cleaved off from an immature protein of total length 93 amino acids) and thus retains the chemokine fold (SEQ ID NO: 10). FmocLys(ivDde)-OH is incorporated as residue 64 to facilitate site-specific labelling at this position of the protein (SEQ ID NO: 11). The naturally occurring lysine at position 64 is modified through biotinylation. The final protein may thus comprise, consist essentially of or consist of the amino acid sequence of SEQ ID NO: 12.

Thus, exemplified herein is a truncated/modified chemokine comprising, consisting essentially of or consisting of the amino acid sequence of SEQ ID NO: 12: X is an amino acid residue that can be biotinylated, such as lysine, ornithine or diaminopropionic acid and optionally is biotinylated, optionally via a spacer molecule such as PEG, especially K(Biotin)

A further example of a chemokine is described in detail herein (see Reference Example 7 below). The modified CCL22 (MDC) corresponds to residues 1 to 69 of the full length mature protein (and lacks the N-terminal signal peptide of 24 amino acids, which is cleaved off) and thus retains the chemokine fold (SEQ ID NO: 13). FmocLys(ivDde)-OH is incorporated as residue 66 to facilitate site-specific labelling at this position of the protein (SEQ ID NO: 14). The naturally occurring lysine at position 66 is modified through biotinylation. A PEG spacer may be incorporated between the ε-amino functionality and the biotin. The final protein may thus comprise, consist essentially of or consist of the amino acid sequence of SEQ ID NO: 15.

Thus, exemplified herein is a modified chemokine comprising, consisting essentially of or consisting of the amino acid sequence of SEQ ID NO: 15: X is an amino acid residue that can be biotinylated, such as lysine, ornithine or diaminopropionic acid and optionally is biotinylated, optionally via a spacer molecule such as PEG, especially K(PEG-Biotin)

A further example of a chemokine is described in detail herein (see Reference Example 8 below). The modified CCL17 (TARC) corresponds to residues 1 to 71 of the full length mature protein (and lacks the N-terminal signal peptide of 23 amino acids, which is cleaved off) and thus retains the chemokine fold. An additional lysine or equivalent residue may be inserted at the C-terminus, at position 72. The chemokine may thus comprise, consist essentially of or consist of the amino acid sequence of SEQ ID NO: 16. FmocLys(ivDde)-OH is incorporated as residue 72 to facilitate site-specific labelling at this position of the protein (SEQ ID NO: 17). The ε-amino side chain functionality of Lys(72) is modified through biotinylation. A PEG spacer may be incorporated between the ε-amino functionality and the biotin. The final protein may thus comprise, consist essentially of or consist of the amino acid sequence of SEQ ID NO: 18.

Thus, it is exemplified herein a modified chemokine comprising, consisting essentially of or consisting of the amino acid sequence of SEQ ID NO: 16 or 18: X is an amino acid residue that can be biotinylated, such as lysine, ornithine or diaminopropionic acid and optionally is biotinylated (e.g. K-biotin), optionally via a spacer molecule such as PEG, in particular K(PEG-Biotin)

A further example of a chemokine of the various embodiments of the invention containing modifications and specifically adapted for use in the invention is described in detail herein (see Example 9 below). The modified CCL19 (MIP-3β) corresponds to residues 1 to 77 of the full length mature protein (and lacks the N-terminal signal peptide of 21 amino acids, which is cleaved off) and thus retains the chemokine fold. An additional lysine is inserted at the C-terminus, at position 78. The chemokine may thus comprise, consist essentially of or consist of the amino acid sequence of SEQ ID NO: 19. FmocLys(ivDde)-OH is incorporated as residue 78 to facilitate site-specific labelling at this position of the protein (SEQ ID NO: 20). The ε-amino side chain functionality of Lys(78) is modified through biotinylation. The final protein may thus comprise, consist essentially of or consist of the amino acid sequence of SEQ ID NO: 21.

Thus, in certain embodiments the invention also relates to a modified chemokine comprising, consisting essentially of or consisting of the amino acid sequence of SEQ ID NO: 19 or 21: X is an amino acid residue that can be biotinylated, such as lysine, ornithine or diaminopropionic acid and optionally is biotinylated (e.g. K-biotin), optionally via a spacer molecule such as PEG, in particular K(PEG-Biotin) X is K(Biotin)

A further example of a chemokine is described in detail herein (see Reference Example 10 below). The modified CXCL11 (ITAC) corresponds to residues 1 to 73 of the full length mature protein (and lacks the N-terminal signal peptide of 21 amino acids, which is cleaved off) and thus retains the chemokine fold (SEQ ID NO: 22). An additional lysine is inserted at the C-terminus, optionally via a PEG spacer, at position 74. The chemokine may thus comprise, consist essentially of or consist of the amino acid sequence of SEQ ID NO: 23. X is an amino acid residue that can be biotinylated, such as lysine, ornithine or diaminopropionic acid and optionally is biotinylated, optionally via a spacer molecule such as PEG. The amino acid residue may be added via a spacer molecule such as PEG and may thus be "PEG-K".

FmocLys(ivDde)-OH is incorporated, following Fmoc-12-amino-4,7,10-trioxadodecanoic acid, as residue 74 to facilitate site-specific labelling at this position of the protein (SEQ ID NO: 24). The ε-amino side chain functionality of the additional Lys(74) is modified through biotinylation. The final protein may thus comprise, consist essentially of or consist of the amino acid sequence of SEQ ID NO: 25. X is PEG-K(biotin).

Thus, exemplified herein is a modified chemokine comprising, consisting essentially of or consisting of the amino acid sequence of SEQ ID NO: 23 or 25.

A further example of a chemokine is described in detail herein (see Reference Example 11 below). The truncated form of human CCL25 (TECK) corresponds to residues 1-74 of the mature protein, which encompasses the sequence corresponding to the chemokine fold. The full length mature protein is 127 amino acids (the signal peptide is 23 amino acids in a 150 amino acid immature protein). The single methionine within the sequence is altered to Norleucine, to mitigate against oxidation of this residue during the chain assembly, which was observed during the synthesis of the natural sequence derivative. The Gln at the N-terminus of the proteins is subject to pyroGlu formation under physiological conditions. Thus Gln1 of the sequence was substituted with pyroglutamine to prevent mixed species of N-terminal Gln and pyroGlu being generated. This improves the yield of synthesis and ensures a homogeneous chemokine preparation through column manufacture and use. The naturally occurring lysine at position 72 was modified through biotinylation on the resin. A PEG spacer was incorporated between the ε-amino functionality and the biotin.

Thus, exemplified herein is a modified chemokine comprising, consisting essentially of or consisting of the amino acid sequence of SEQ ID NO: 26:
X1 = pyroGlu or Gln
X64 = Norleucine
X72 = an amino acid residue that can be biotinylated, such as lysine, ornithine or diaminopropionic acid and optionally is biotinylated, optionally via a spacer molecule such as PEG, e.g. K(PEG-Biotin)

X1 = pyroGlu or Gln
X64 = Norleucine
X72 = K(ivDde)

FmocLys(ivDde)-OH may be incorporated as residue 72 to facilitate site-specific labelling at this position of the protein (SEQ ID NO: 27). Subsequent removal of the ivDde protecting group, followed by coupling of the PEG spacer and Biotin, may be carried out as described in the general protocol section. The desired active chemokine may thus comprise, consist essentially of or consist of the amino acid sequence of SEQ ID NO: 28:
X1 = pyroGlu or Gln
X64 = Norleucine
X72 is K(PEG-Biotin)

A further example of a chemokine is described in detail herein (see Reference Example 12 below). Human CCL27 (CTAC) corresponding to residues 1-88, is initially expressed as 112 amino acids comprising the chemokine fold, and a 24 amino acid signal peptide which is cleaved off. The Met(87) within the sequence was mutated to lysine to provide a lysine at position 87 which was modified through biotinylation on the resin. A PEG spacer was incorporated between the ε-amino functionality and the biotin.

Thus, exemplified herein is a modified chemokine comprising, consisting essentially of or consisting of the amino acid sequence of SEQ ID NO: 29: X is an amino acid residue that can be biotinylated, such as lysine, ornithine or diaminopropionic acid and optionally is biotinylated, optionally via a spacer molecule such as PEG, e.g. K(PEG-Biotin) X = K(ivDde)

FmocLys(ivDde)-OH is incorporated as residue 87 to facilitate site-specific labelling at this position of the protein (SEQ ID NO: 30). Subsequent removal of the ivDde protecting group, followed by coupling of the PEG spacer and Biotin, was carried out as described in the general protocol section. The desired active chemokine may thus comprise, consist essentially of or consist of the amino acid sequence of SEQ ID NO: 31: X = K(PEG-Biotin)

A further example of a chemokine is described in detail herein (see Example 13 below). The modified CXCL10 (IP-10) corresponds to residues 1 to 78 of the full length mature protein (and lacks the N-terminal signal peptide of 21 amino acids, which is cleaved off) and thus retains the chemokine fold. An amino acid which is capable of biotinylation, such as lysine or ornithine for example, may be inserted as residue 78. Insertion may be via a spacer, such as a PEG spacer. The linear amino acid sequence (SEQ ID NO: 32) is shown, prior to attachment of the PEG spacer, additional lysine and biotin molecules:

Thus, position 78 may be modified through biotinylation. FmocLys(ivDde)-OH may be incorporated as residue 78 to facilitate site-specific labelling at this position of the protein (SEQ ID NO: 33). A suitable spacer, such as a PEG spacer, may be incorporated between the ε-amino functionality and the biotin. The biotinylated version comprises, consists essentially of or consists of the amino acid sequence of SEQ ID NO: 34.

Thus, exemplified herein is a modified chemokine comprising, consisting essentially of or consisting of the amino acid sequence of SEQ ID NO: 34: X is an amino acid residue that can be biotinylated, such as lysine, ornithine or diaminopropionic acid and optionally is biotinylated, optionally via a spacer molecule such as PEG, e.g. K(PEG-Biotin) and may be attached via a spacer molecule, e.g.PEG-K(Biotin).

Chemokines useful in the various embodiments of the invention may be synthesised through any suitable means known in the art. Preferably, the chemokines are chemically synthesised as this facilitates modification and labelling etc. However, recombinant DNA based approaches may also be employed in combination with appropriate labelling and modification technologies as required. As indicated above, site-specific labelling of the chemokines of the various embodiments of the invention is preferable, although any labelling technique which does not significantly affect the receptor-binding capacity of the chemokine may be employed. Various site-specifically biotinylated chemokines and native chemokines are available commercially, for instance from Almac, Craigavon, UK. In specific embodiments the one or more chemokines are biotinylated via a spacer group. The spacer may be employed to prevent the biotin group from impacting on the activity of the chemokine, in particular binding of the chemokine to its cognate receptor. Any suitable spacer that facilitates retention of receptor binding properties of the chemokine may be employed in the various embodiments of the invention. Thus, in the specific embodiments described above, spacers other than PEG spacers may be employed as appropriate. In specific embodiments, the spacer is a polyethylene glycol (PEG) spacer. PEG has been shown to be an effective spacer permitting attachment of biotin to the chemokine (which can then be immobilized on the solid support through interaction with streptavidin) without compromising receptor binding capability. In the context of the various embodiments of the present invention the term "antibody" includes all immunoglobulins or immunoglobulin-like molecules with specific binding affinity for the relevant chemokine receptor (including by way of example and without limitation, IgA, IgD, IgE, IgG and IgM, combinations thereof, and similar molecules produced during an immune response in any vertebrate, for example, in mammals such as humans, goats, rabbits and mice).

**Table 1. Commercially available fluorophore labelled antibodies against specific chemokine receptors.**

| **Antibody** | **Fluorophore** | **Supplier** |
|---|---|---|
| CCR5 | PE | Biolegend |
| CXCR7 | PE | Biolegend |
| CCR6 | PE | BD Biosciences |
| CCR4 | PerCP Cy5.5 | BD Biosciences |
| CCR7 | PerCP Cy5.5 | Biolegend |
| CCR6 | PerCP Cy5.5 | BD Biosciences |
| CXCR4 | APC | R&D Systems |
| CCR8 | APC | R&D Systems |

Thus, in certain embodiments the invention also enables an apheresis column loaded with a solid support comprising a binding reagent capable of specifically binding to a chemokine receptor immobilized directly or indirectly on the support to permit removal of a cell expressing the chemokine receptor from the peripheral blood of a patient/subject, wherein the binding reagent is not a chemokine. The binding reagent capable of specifically binding to the chemokine receptor may be an agonist or an antagonist of the chemokine receptor. In specific embodiments, the binding reagent capable of specifically binding to the chemokine receptor is selected from an antibody and a chemical compound.

Solid support materials for immobilizing the binding reagents of the various embodiments of the invention are known in the art. "Solid support" refers to, for example, materials having a rigid or semi-rigid surface or surfaces, and may take the form of beads, resins, gels, microspheres, or other geometric configurations. A useful support material is one that does not activate blood cells so as to make them coagulate or adhere to the support as peripheral whole blood is applied to the device. In certain embodiments, a support treated with an agent to provide it with anti-coagulation properties, in particular a heparinized support is employed. Alternatively, the blood of the patient may be treated with an anti-coagulant such as heparin prior to application to the support. Useful support materials comprise high molecular weight carbohydrates, in particular carbohydrates having a molecular weight of 100 kDa or more, such as agarose, in particulate form, optionally cross-linked, and cellulose. Other preferred support materials are polymers, such as carboxylated polystyrene, and glass. The support of the various embodiments of the invention may be provided in the form of particles or fibres. The support particles may have regular form, such as spheres or beads, or irregular form. They may be porous or non-porous. A preferred average particle size of the support is from 50 µm to 2 mm. In certain embodiments Sepharose™, a cross linked, beaded-form of agarose, is used as the column matrix. It is chosen for its optimal distribution capacity and can provide a large available area for affinity binding. Solid supports may be provided in the form of magnetic beads, with the specific binding reagent immobilized on the magnetic beads. Following capture of the CCR7 chemokine receptor receptor expressing cells from the blood, the beads can be removed from the blood with the aid of an appropriate magnetic separator.

Methods for immobilizing binding reagents on a solid support are known in the art. A binding reagent, such as a chemokine can be immobilized on the support in a direct or indirect manner. Immobilization can be by means of a suitable linker in some embodiments. A preferred method of indirect immobilization of a binding reagent, such as a chemokine, relies upon the interaction between biotin and avidin molecules. "Avidin" or "avidin molecule" refers to any type of protein that specifically binds biotin to the substantial exclusion of other (small) molecules that might be present in a biological sample. Examples of avidin include avidins that are naturally present in egg white, oilseed protein (e.g., soybean meal), and grain (e.g., corn/maize), and streptavidin, which is a protein of bacterial origin. Thus, biotinylation of the binding reagent and use of an avidin molecule such as streptavidin immobilized on the solid support allows reliable attachment of the binding reagent to the solid support according to methods known in the art. Specifically, such a method may comprise providing the binding reagent in biotinylated form, providing a solid support having streptavidin immobilized on its surface, contacting the support with an aqueous solution of the biotinylated binding reagent, and rinsing the support with an aqueous solvent. In addition, binding pair interactions, such as antibody - antigen interactions, may also be utilised for indirect immobilisation of binding reagent onto a support. In such embodiments the support may be derivatised with one member of a binding pair, such as an antibody or fragment or derivative thereof, as defined herein, which has known affinity for a particular peptide sequence or small molecule hapten. Incorporating the other member of the binding pair, such as an antigen, peptide sequence or the hapten onto or into the binding reagent facilitates immobilisation onto a solid support coated with the corresponding antibody or fragment or derivative thereof. Thus, the binding reagent may be modified to include the peptide sequence or hapten into the linear molecule or may be added as a side chain or label. Any suitable antibody-antigen pair may be employed. The antibody fragment or derivative may be any fragment or derivative that retains specific binding affinity for the appropriate antigen. Examples include Fab, F(ab')₂ fragments, scFV, VH domains, single domain antibodies (such as nanobodies), heavy chain antibodies and humanized version of non-human antibodies etc. Other high affinity interactions can be utilised for immobilisation of binding reagents, as long as the binding reagent can be synthesised or derivatised with one of the interacting partners and the solid support synthesised or derivatised with the other interacting partner without loss of binding activity (i.e. binding of the binding reagent to the appropriate chemokine receptor). Immobilization may occur via essentially the same interaction in reverse in some embodiments. Thus, the binding reagent which may be a chemokine for example, may be attached to an antibody as defined herein, and the solid support derivatised with the antigen. The chemokine may be produced as a fusion protein with the antibody.

Alternatively binding reagents, such as chemokines, can be immobilised directly onto a solid support using bioconjugation techniques established in the field. For example direct immobilisation of proteins onto cyanogen bromide activated solid supports via amino functionalities within the primary sequence of the protein. Alternatively, sulphydryl functionalities within proteins can be used to directly immobilise the protein to alkyl halide derivatised supports or supports containing free thiol functionalities. In further embodiments, proteins containing α-thioester functionalities can be directly immobilised on supports containing 1,2 amino thiol moieties (eg N-terminal cysteine) using the native chemical ligation reaction. Alternatively proteins modified with ketones and aldehydes can be immobilised on solid supports derivatised with hydrazinyl, hydrazide and aminoxy functionalities using hydrazone / oxime bond forming ligation reactions (and vice versa). Alternatively 'Click' chemistry can be used to immobilise proteins onto solid supports, whereby the protein and the support are derivatised with the appropriate mutually reactive chemical functionalities (azides and alkynes). In other embodiments Staudinger ligation chemistry can be used to immobilise appropriately derivatised proteins onto the appropriately derivatised solid supports.

The solid support is contained within or carried by the apheresis column. Thus, by "loaded" is meant that the column carries or contains the solid support in a manner such that (peripheral) blood can flow through the column in contact with the solid support. Thus, the solid support provides a matrix within the column through which blood flows, in continuous fashion in certain embodiments. This permits cells expressing the specific chemokine receptor to be removed from the blood passing through the column, such that blood exiting the column is depleted of the specific chemokine receptor-expressing cells. In specific embodiments, the apheresis column is loaded with a support comprising streptavidin immobilized on the support and one or more biotinylated binding reagents, such as chemokines, bound to the streptavidin on the support. The solid support may be comprised of a high-molecular weight carbohydrate, optionally cross-linked, such as agarose.

As discussed above, the binding reagent is coupled to the solid support. The relative amounts of binding reagent may be controlled to ensure that coupling between the solid support and the binding reagent will be immediate, minimising the risk of binding reagent decoupling from the solid support. Thus, it may be ensured that there is a relative excess of immobilization sites for the binding reagent on the solid support. Alternatively, or additionally, following immobilization of the binding reagent on the solid support, the solid support may be washed to remove any unbound binding reagent.

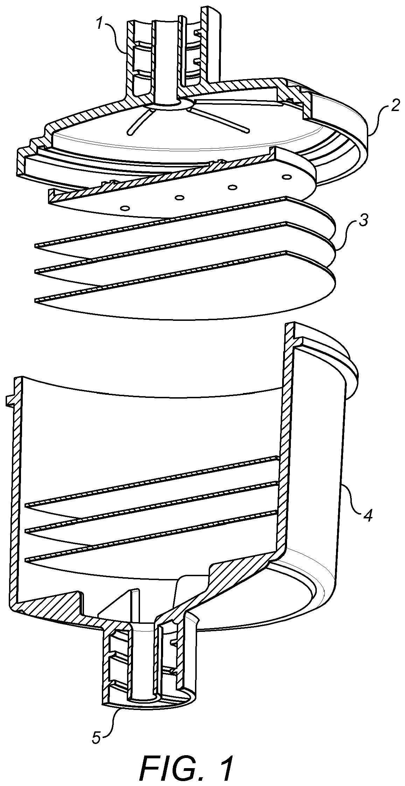

The apheresis column utilised in the various embodiments of the present invention acts as a leukapheresis treatment for cancer. The column acts to specifically remove CCR7, by exploiting the interaction between CCR7 expressed on the cell surface and a specific binding reagent immobilized on a solid support contained within or carried by the column. In other embodiments, the column acts to specifically remove one or more of Treg receptor expressing cells The overall column typically comprises, consists of, or consists essentially of three combined components; 1) a housing which contains or carries 2) the solid support and 3) one or more binding reagents (immobilized thereon) which specifically bind one or more chemokine receptors. The housing may be manufactured from any suitable material for clinical use. In certain embodiments the housing is composed of a plastic material. The housing includes an in flow site for entry of blood and an out flow site for blood (depleted of target cells) to exit the column. The housing may be designed to maintain a continuous blood flow through the solid support matrix. The housing (as shown for example in Figure 1) may include a top portion which comprises a distribution plate (2) at the inflow site (1) to spread the blood evenly over the entire matrix area. The distribution plate may act as a first safety barrier preventing larger particles flowing through the column and into the patient. However, the distribution plate is not essential and may be removed in some embodiments to decrease the overall resistance in the system. The column may contain one or more safety filter units (3 and 4) placed at the inflow (1) and/or outflow (5) sites of the plastic housing. Such filter units may act to prevent particles larger than blood cells passing in and/or out of the column. The safety filter units may contain a plurality of filters, such as two, three or four filters designed to be a robust barrier and stop all particles larger than blood cells passing through the column. Inclusion of safety filters (3 and 4) at both ends of the column serves to minimize the risk of leakage of particles into the patient, including in the event that the device is incorrectly connected resulting in blood flow in the opposite direction to that intended. The safety filters may comprise of any suitable pore size to prevent particles larger than blood cells from passing through the column, as would be readily understood by one skilled in the art. Suitable filters are commercially available. In specific embodiments, the pore size of the filter(s) is between approximately 60µm and 100µm, more specifically approximately 80 µm. The solid support and binding reagent components are discussed in further detail herein. The volume of the housing may be varied depending upon the blood volumes intended to pass through the column. Typically, the volume of the housing is between approximately 40ml and 200ml, more specifically 50ml to 150ml or 60ml to 120ml. For leukemia treatments housing volumes tend to be higher, due to the large number of cells which are to be removed from the blood. In such embodiments, the housing volume is typically in the region of approximately 100ml to 500ml, such as 100ml to 300ml or 120ml to 150ml. In specific embodiments, the housing volume is approximately 120ml.

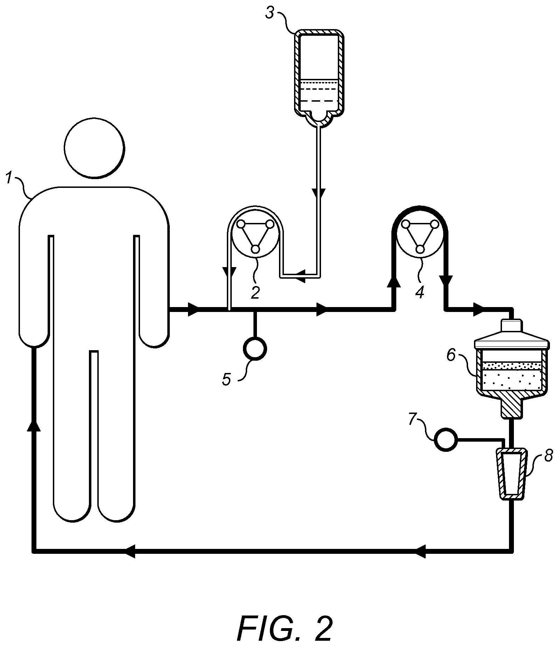

The column is generally applied in the form of an apheresis circuit. In this context, the overall system includes the apheresis column, tubing and an appropriate pump to pump the blood around the circuit. The system is illustrated in figure 2. The patient (1) is connected to the extracorporeal circuit via sterile needles to veins in the right and the left arms. A saline bag (3) is also connected and the saline solution is pumped with a suitable pump (2). Blood is drawn from one arm of the patient through the sterile tubing system by the blood pump (4) and passed through the column (6) and back to the patient. The tubing system may be connected to the column via any suitable coupling, such as standard dialysis luer-lock couplings. The couplings on the column may be colour-coded for correct assembly. For example, red tubing for inflow to the red column top and blue tubing for outflow back to the patient. An air detector (8) may be present in the circuit. Inlet pressure (5) and/or Pven sensors (7) may additionally be employed to monitor the pressure in the circuit.

An apheresis pump, such as the 4008 ADS pump manufactured by Fresenius Medical Care or the Adamonitor pump, may monitor the patient's inflow and outflow. The pump may also monitor the pressure in the extracorporeal circulation. The pump may be able to discriminate air by a bubble catcher and air detector. A clot catcher filter may be positioned inside the bubble catcher. The pump may also incorporate an optical detector to distinguish between light, e.g. saline solution or air present in the tubing system and dark e.g. blood present in the tubing system.

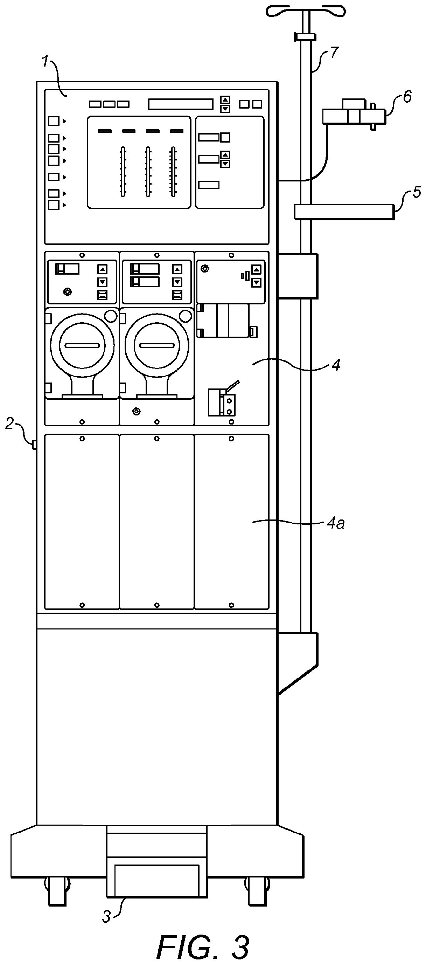

A schematic diagram of a suitable pump, showing the air detector and optical filter is shown in figure 3. If the pump system detects air bubbles and optical fluctuations or if extracorporeal pressure values are out of the set range, then the pump may stop immediately. Alternatively or additionally a visual/ audible alarm may be emitted.

In present text, leukemias such as CLL are diagnosed on the basis of levels of CCR7 expressing cells. A positive diagnosis may be made in subjects where the levels of CCR7 expressing cells are sufficient to interfere with normal hematopoiesis. This may be when the majority of circulating cells are leukemic, and in addition are CCR7 positive. Thus, diagnosis may be made based upon the presence of greater than about 50%, such as greater than about 55%, greater than about 60%, greater than about 65%, greater than about 70%, greater than about 75%, greater than about 80%, greater than about 85%, greater than about 90%, greater than about 95% or more CCR7 expressing cells in the sample, as a percentage of total cells in the sample.

The various embodiments of the invention will now be described in more detail:

### DESCRIPTION OF THE FIGURES

FIG. 1 - The plastic house and top showing the distribution plate (2) and safety filter units (3 and 4).
FIG. 2 - The overall leukapheresis system.
FIG. 3 - The pump with air detector and optical detector (4).
FIG. 4 - Results of in vitro depletion tests performed on the biotinylated MIP-3alpha coupled matrix showing ability to eliminate CCR6-expressing lymphocytes from blood from three healthy donors.
FIG. 5 - Example of gating criteria for CCR2 expressing monocytes
FIG. 6 - Percentage of different leukocyte populations in a patient with Chronic Lymphatic Leukemia and in a healthy control (HC). Blood from a patient with CLL and a healthy control were analysed for the expression of cell specific markers with flow cytometry. The B cells were characterized as CD19 positive, the T cells as CD3 positive, granulocytes as CD16 positive and monocytes as CD14 positive.
FIG. 7 - Expression of CCR7 on B cells from a patient with Chronic Lymphatic Leukemia. Blood from a patient with CCL was analysed for the expression of various chemokine receptors by flow cytometry. The B cells were characterized as CD19+ lymphocytes.
FIG. 8 - Binding of the biotinylated chemokine MIP3b (CCL19) to B cells from a patient with Chronic Lymphatic Leukemia. Blood from a patient with CLL was incubated with bMIP3b and analysed by flow cytometry. The B cells were characterized as CD19+ lymphocytes.
FIG. 9 - Depletion of B cells with Sepharose Streptavidin matrix conjugated with bMIP3b. Blood cells from a patient with CLL were incubated with bMIP3b- SepharoseStreptavidin matrix. Unbound cells were retrieved by washing the matrix with Phosphate Buffer Saline. The cells (After Depletion) were then analysed by flow cytometry and compared with cells that had not been incubated with the MIP3b-matrix (Before Depletion).
FIG. 10 - Frequency of CCR4 positive Tregs in eight healthy controls (HC), two patients with Pancreatic Cancer (PC) and one patient with Urinay Bladder Cancer (UBC). The expression of chemokine receptors and specific cell markers was analyzed with flow cytometry. The Tregs were defined as CD4 positive, CD25 positive (hi), CD127 negative cells.
FIG. 11 - CCR4 expression on Tregs compared to T cells in UBC (left) and PC (right). The expression of chemokine receptors and specific cell markers was analysed with flow cytometry in two patients with PC and one patient with UBC.
FIG. 12 - Binding of the chemokine bMDC to Tregs. Blood cells from a patient with PC were incubated with biotinylated chemokine or an anti-CCR4 antibody and analyzed with flow cytometry.
FIG. 13 - Depletion of CCR4 expressing T cells with Sepharose Streptavidin-matrix conjugated with biotinylated MDC (bMDC). Blood cells from a patient with UBC were incubated with biotinylated chemokine-Sepharose Streptavidin-matrix. Unbound cells were retrieved by washing the matrix. The unbound cells (After Depletion) were then analysed with flow cytometry and compared with cells that had not been incubated with bchemokine-matrix (Before Depletion).

### DESCRIPTION OF PREFERRED EMBODIMENTS

The mechanism behind seeding of metastatic cells in to particular organs is by the expression of chemokines in the organ recruiting chemokine receptor expressing tumor cells. For example metastatic colon cancer cells express CCR6 and they home to liver because CCL20 is expressed in the liver allowing entrance of CCR6 expressing cancer cells.

In non Hodgkin lymphomas (T-NHL) the expression of CCR7 indicates a higher lymphatic dissemination which was demonstrated to migrate towards CCL21, the preferred chemokine for entrance of cells in to lymphoid organs.
Lung cancer metastases seem to be dependent on the expression of CXCR4 on tumour cells and the local expression of the corresponding chemokine CXCL12 (SDF-1) for the successful metastasis. In line with these findings development of small molecules for inhibition of CXCR4 mediated metastasis is under development.
Regulatory T cells (Tregs) are upregulated in many cancers as an immune escape mechanism induced by the tumor. In order to avoid immune recognition and elimination tumors activate and recruit Tregs. The production of chemokines by the tumor results in recruitment of circulating Tregs and therefore protection against immune recognition and elimination. Tregs may be recruited to the tumour by CCL17 and CCL22 binding to CCR4 expressed on the Treg. In addition CCR8 mediated recruitment by CCL1 may occur. Since many treatment regimes with chemotherapy involve activation of the immune system, eliminating Tregs in the circulation by leukapheresis may thus favour immune recognition and elimination of tumor cells, thereby enhancing cancer therapy.

In chronic leukemic disorders the number of circulating tumor cells inhibit normal hematopoeis and therefore cause symptoms. The increased production of chemokines such as CCL3 correlates with poor prognosis (Blood. 2011 Feb 3;117(5):1662-9. Epub 2010 Nov 29). Thus elimination of chemokine receptor expressing leukemic cells will favorably affect the disease in terms of symptoms and disease progression.

It is shown herein that cancer patients, in particular subjects suffering from UBC and PC, exhibit an increased frequency of CCR4 expressing circulating Tregs and that this response is specific to Tregs (and does not apply to other T lymphocytes). CCR4 expressing cells may be thus be targeted in order to treat cancer. Treatment may rely upon suitable binding reagents such as CCL22 (MDC) and derivatives thereof, as described herein in further detail. It is also shown herein that subject suffering from leukemias, such as CLL, have a highly increased number of circulating B cells. The B cells express characteristic chemokine receptors, such as CCR7. It is also shown herein that CCR7 expressing B cells may be efficiently depleted using MIP3b as a specific binding reagent in a leukapheresis method.

### REFERENCE EXAMPLE 1 - Tailored leukapheresis

### COLUMN DESIGN AND PROPERTIES

### Introduction

Apheresis is an established treatment used for depletion of blood components, such as antibodies, low-density lipoproteins (LDL) and blood cells. Leukapheresis is the apheresis treatment used for removal of white blood cells, leukocytes. The patient is connected to an extracorporeal blood circulating system; the blood is drawn from a vein in one arm, passed through a column device and returned into the other arm of the patient. Side effects of leukapheresis treatments are varying from mild events like headache, dizziness, hypotension, palpitation and flush seen in 0.1 to 5% of treated patients.

### The column

The column is intended to be used as a leukapheresis treatment for cancer. It will specifically remove CCR5, CCR6, CCR7, CCR8, CXCR4, CXCR7, CCR4, CCR9, CCR10, CXCR3 and/or CXCR5 -expressing cells, such as tumour cells and leukocytes, such as regulatory T lymphocytes, through the use of a binding reagent, more specifically an MIP-3alpha (CCL20), CCL19, CCL21, CCL1, CXCL11, CXCL12, CCL25 (TECK), CCL27 (CTACK), CCL28 (MEC), CXCL9 (MIG), CXCL10 (IP10), CXCL13 (BCA-1), CCL17 (TARC) and CCL22 (MDC) containing resin, exploiting the CCR5, CCR6, CCR7, CCR8, CXCR4, CXCR7, CCR4, CCR9, CCR10, CXCR3 and/or CXCR5 -chemokine interaction. Treg receptor expressing cells, such as CLA receptor, CCR4 or CCR8 expressing cells may be specifically removed through the use of an appropriate binding reagent, more specifically an SELE, CCL17, CCL22 and/or CCL1 containing resin. The column consists of three combined components, the plastic house, the streptavidin (SA) Sepharose™ BigBeads matrix and one or more of biotinylated MIP-3alpha (CCL20), CCL19, CCL21, CCL1, CXCL11, CXCL12, CCL25 (TECK), CCL27 (CTACK), CCL28 (MEC), CXCL9 (MIG), CXCL10 (IP10), CXCL13 (BCA-1), CCL17 (TARC) and CCL22 (MDC) and/or SELE, CCL17, CCL22 and/or CCL1 bound to the matrix. The treatment is conducted using the same techniques as a standard apheresis procedure.

### The plastic house (FIG. 1)

The plastic house, designed to keep a continuous blood flow through the matrix, consists of a transparent body and red-coloured top. The top has a distribution plate (2) at the inflow site (1) to spread the blood evenly over the entire matrix area. The plate is the first safety barrier preventing larger particles flowing through the column and into the patient. Safety filter units (3 and 4) are placed at the inflow (1) and outflow (5) sites of the plastic housing. The safety filter unit contains three filters designed to be a robust barrier and stop all particles larger than blood cells passing through the column. The plastic housing design is shown in Figure 1. The design with safety filters (3 and 4) at both ends of the column device will minimize the risk of leakage of particles into the patient, including in the event that the device is placed up side down with the blood flow in the opposite direction to that anticipated.

### Streptavidin Sepharose™ BigBeads

The second component in the device is the affinity matrix called streptavidin Sepharose™ BigBeads (Sepharose™ GE Healthcare, Sweden). Sepharose™ is a cross linked, beaded-form of agarose, which is a polysaccharide extracted from seaweed. Sepharose™ and agarose are commonly used as column matrices in biomedical affinity techniques. It is chosen for its optimal distribution capacity and can provide a large available area for affinity binding.

### Binding reagent

Coupled to the matrix is the third component of the device, one or more binding reagents that bind specifically to CCR5, CCR6, CCR7, CCR8, CXCR4, CXCR7, CCR4, CCR9, CCR10, CXCR3 and/or CXCR5 and/or to CLA receptor, CCR4 and/or CCR8 where Tregs are specifically targeted. One or more chemokines selected from the group consisting of: MIP-3alpha (CCL20), CCL19, CCL21, CCL1, CXCL11, CXCL12, CCL25 (TECK), CCL27 (CTACK), CCL28 (MEC), CXCL9 (MIG), CXCL10 (IP10), CXCL13 (BCA-1), CCL17 (TARC) and CCL22 (MDC) may be employed. Alternatively, SELE, CCL17, CCL22 and/or CCL1 may be employed to target Tregs. These peptides may be synthetic, engineered versions of the human chemokine, which are truncated and biotinylated, but retain binding activity to the CCR5, CCR6, CCR7, CCR8, CXCR4, CXCR7, CCR4, CCR9, CCR10, CXCR3 and/or CXCR5 receptor (or CLA receptor, CCR4 and/or CCR8 where Tregs are targeted). By biotinylating the engineered chemokine, it is able to bind to the streptavidin molecules in the Sepharose™ matrix. The biotin-streptavidin binding is known be one of the strongest biological interactions with a Kd in the order of 4 x 10⁻¹⁴ M. The calculated ratio of streptavidin:biotin binding sites in the column is 10:1. Therefore, the coupling between the matrix and chemokine will be immediate, minimising the risk of chemokine decoupling from the matrix.

### The apheresis system

To conduct the leukapheresis the following components are needed; the column, tubing system, and a 4008 ADS pump (Fresenius Medical Care).

### The circuit

The system is illustrated in Figure 2. The patient (1) is connected to the extracorporeal circuit via sterile Venflon needles to veins in the right and the left arms. A saline bag (3) is also connected and the saline solution is pumped with an ACD pump (2). Blood is drawn from one arm of the patient through the sterile tubing system by the blood pump (4) and passed through the column (6) and back to the patient. The tubing system is connected to the column via standard dialysis luer-lock couplings. The couplings on the column are colour-coded for correct assembly; red tubing for inflow to the red column top and blue tubing for outflow back to the patient. An air detector (8) is present. Inlet pressure (5) and Pven sensors (7) are employed to monitor the pressure in the circuit.

### The 4008 ADS pump

An apheresis pump, from Fresenius Medical Care, monitors the patient's inflow and outflow, the pressure in the extracorporeal circulation and can discriminate air by a bubble catcher and air detector. A clot catcher filter is placed inside the bubble catcher. The pump also has an optical detector to distinguish between light, e.g. saline solution or air present in the tubing system and dark e.g. blood present in the tubing system.
A schematic diagram of the pump, showing the air detector and optical filter is shown in Figure 3. If the pump system detects air bubbles and optical fluctuations or if extracorporeal pressure values are out of the set range, then the pump stops immediately and a visual/ audible alarm are emitted.

Legend for FIG. 3:
1. Monitor
2. Holder for waste bag
3. Modules (left to right - Blood pump, ACD pump, Air detector)
4. Reserve places for further modules
5. Absorber holder
6. Drip detector
7. IV pole

### Preparation of the patient

The patient will be administered anticoagulants prior to each treatment session. A sterile saline solution with 5000 IE Heparin will be used for priming the extracorporeal system, thereafter a bolus injection with 4000 IE Heparin will be added into the circuit at the start of each treatment session.

### Leukapheresis time and flow rate

The apheresis system should be operated at a flow rate of 30-60 mL/min. A treatment is finalised after 1800mL of blood has been circulated.

### Storage conditions

The column devices should be stored between 1 and 25°C avoiding freezing and more elevated temperatures. Stability data > 3 months indicate no difference in functionality over time or by temperature (room temperature and refrigerated). The columns will be kept in refrigerated conditions until use. Mechanical damage as those resulting from violent vibrations and trauma should be avoided. Column stored outside of these recommendations should not be used.

### Transport conditions

The column devices will be transported under refrigerated condition, avoiding freezing and more elevated temperatures. Mechanical damage such as those resulting from violent vibrations and trauma should be avoided.

### In-vitro depletion of target cell populations

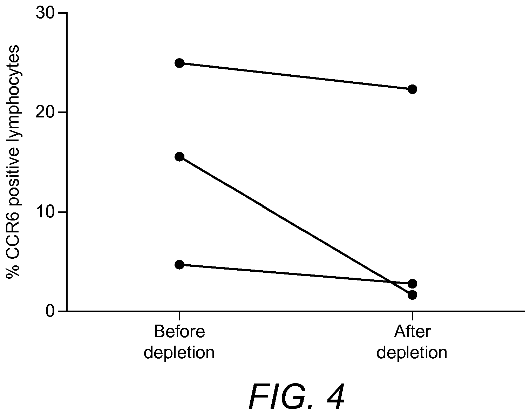

To investigate the ability to eliminate CCR6-expressing cells, in vitro tests have been performed on the biotinylated MIP-3alpha coupled matrix. Blood was collected from blood donors and passed through the column device containing biotinylated MIP-3alpha coupled matrix. Blood samples were taken before and after column passage and analyzed by flow cytometry (FACS) for the depletion of CCR6-expressing cells.
The results demonstrate significant depletion of the target population CCR6-expressing lymphocytes post matrix perfusion. Depletion tests were performed on blood from three healthy donors. The results are shown in Figure 4.
The in-vitro results demonstrate a specific reduction of up to around 15% of the CCR6-expressing cells by the column. Non-CCR6-expressing cells remained unaffected (data not shown).

### REFERENCE Example 2A - Treatment of Chronic Lymphatic Leukemia (CLL) patient

### Materials and methods

### 1. Flow cytometric analysis of peripheral blood

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH4CI, pH 7.5) for 15 min at 37°C. The cells were washed in PBS with 2% Bovine Growth Serum, incubated with 10% human serum for 15 min at room temperature (RT) and stained with antibodies (Table 2) at 4°C for 30min. The cells were analysed by flow cytometry on a FACS Canto flow cytometer with the FACSDiva software (BD Biosciences).

**Table 2. List of antibodies for flow cytometric analysis.**

| **Antibody** | **Fluorophore** | **Supplier** |
|---|---|---|
| CD14 | FITC | Beckman Coulter |
| Streptavidin | PE, APC | Biolegend |
| CCR7 | PerCP Cy5.5 | Biolegend |
| CD16 | PE Cy7 | BD Biosciences |
| CD3 | V450 | BD Biosciences |
| CD19 | V500 | BD Biosciences |

### 2. Chemokine Binding test

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH4CI, pH 7.5) for 15 min at 37°C. The cells were washed in PBS with 2% Bovine Growth Serum, incubated with 10% human serum for 15 min at room temperature (RT) and stained with cell specific antibodies together with biotinylated chemokine (1 µM) or the corresponding chemokine receptor antibody at 4°C for 30min (Table 2). The biotinylated chemokine was detected via the interaction between biotin and a fluorophore conjugated Streptavidin. The samples were analysed by flow cytometry on a FACS Canto flow cytometer with the FACSDiva software (BD Biosciences).

### 3. Cell depletion by matrix conjugated with biotinylated chemokine

Cells were prepared from peripheral blood (section 1). 1 mL Sepharose BigBeads matrix conjugated with 0.4mg/mL Streptavidin (GE Healthcare) was washed in 50 mL PBS and added to a 5 mL polystyrene tube (BD Falcon™). Biotinylated chemokine (1µM) was added to the tube and incubated for 20 min at RT to enable immobilization of the chemokine on the matrix via the biotin-streptavidin interaction. Next, the cells were added to the chemokine-matrix and incubated for 20 min at RT. The cells that did not bind to the matrix were removed by washing the matrix with PBS in a sterile 40µm nylon filter (BD Falcon™ Cell Strainer). The flow through cells were stained with antibodies (Table 2), analysed by flow cytometry and compared with cells from peripheral blood that had not been incubated with the chemokine-matrix.

### Results and Discussion

### 1. Flow cytometric analysis of peripheral blood

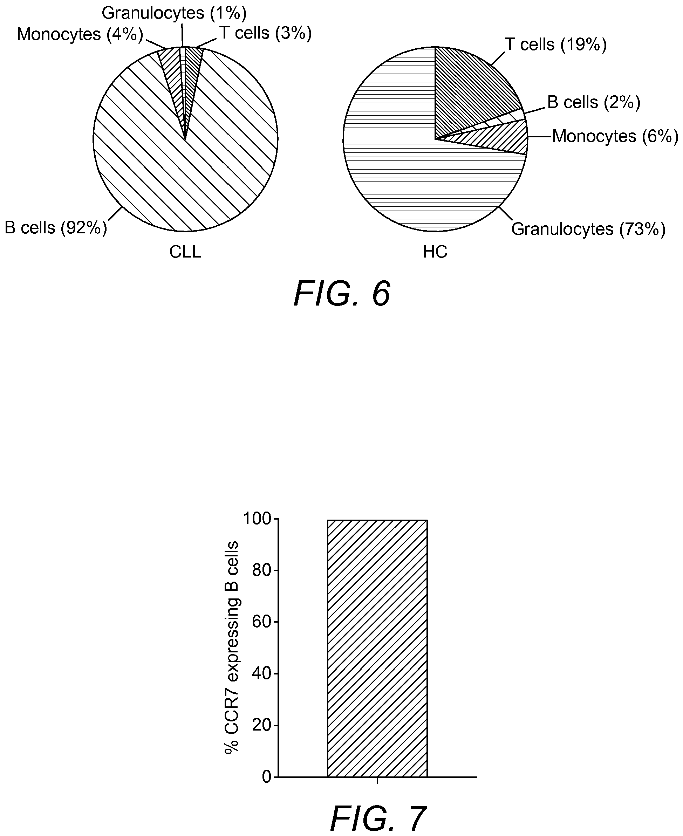

White blood cells from a CLL patient were analysed by flow cytometry. The patient had a highly increased number of circulating B cells; 92% compared to approximately 2% in healthy blood (FIG. 6). This finding is common in CLL where malignant B cells undergo extensive proliferation, accumulate in the bone marrow and blood and crowd out healthy blood cells.

### 2. Chemokine Binding test

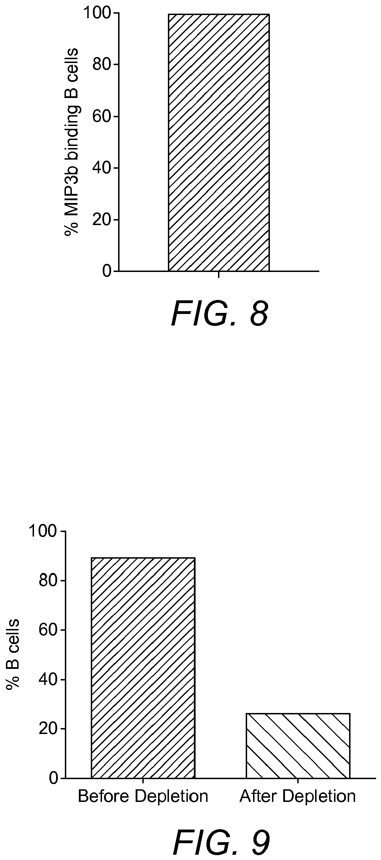

All the B cells were shown to express the chemokine receptor CCR7 (FIG. 7) based upon flow cytometry data and binding by an anti-CCR7 antibody. CCR7 is important for lymph node homing which is mediated by binding to chemokines such as MIP3b (CCL19) and SLC (CCL21) that are expressed in lymphoid tissue. In accordance with the CCR7 expression, all the B cells bound to a biotinylated MIP3b (bMIP3b) (FIG. 8).

### 3. Cell depletion by matrix conjugated with biotinylated chemokine

The B cells were efficiently depleted using bMIP3b-conjugated Sepharose Streptavidin Matrix. Before depletion the B cells constituted 89.1% of the cells and after depletion 26.4% (FIG. 9).

We conclude that B cells in CLL express CCR7 and bind the ligand bMIP3b. Furthermore the majority (62.7%) of the CCR7 expressing B cells can be removed using a Sepharose Streptavidin matrix conjugated with bMIP3b.

### REFERENCE Example 2B - Treatment of Cancers via Removal of CCR4 expressing Tregs using biotinylated-MDC (CCL22)

### Materials and methods

### 1. Flow cytometric analysis of peripheral blood

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH₄Cl, pH 7.5) for 15 min at 37°C. The cells were washed in PBS with 2% Bovine Growth Serum, incubated with 10% human serum for 15min at room temperature (RT) and stained with antibodies (Table 3) at 4°C for 30min. The cells were analysed by flow cytometry on a FACS Canto flow cytometer with the FACSDiva software (BD Biosciences).

**Table 3. List of antibodies for flow cytometric analysis.**

| **Antibody** | **Fluorophore** | **Supplier** |
|---|---|---|
| CCR4 | PerCP Cy5.5 | BD Biosciences |
| CD127 | PE Cy7 | Biolegend |
| CD4 | V500 | Biolegend |
| CD25 | APCCy7 | Biolegend |
| CD3 | Pacific blue | BD Biosciences |
| Streptavdin | PerCpCy5.5 | BD Biosciences |
| CD25 | FITC | BD Biosciences |

### 2. Chemokine Binding test

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH4CI, pH 7.5) for 15 min at 37°C. The cells were washed in PBS with 2% Bovine Growth Serum, incubated with 10% human serum 15min at room temperature (RT) and stained with cell specific antibodies together with biotinylated chemokine (1µM) or the corresponding chemokine receptor antibody at 4°C for 30min (Table 3). The biotinylated chemokine was detected via the interaction between biotin and a fluorophore conjugated Streptavidin. The samples were analysed by flow cytometry on a FACS Canto flow cytometer with the FACSDiva software (BD Biosciences).

### 3. Cell depletion by matrix conjugated with biotinylated chemokine

Cells were prepared from peripheral blood (section 1). 1 mL Sepharose BigBeads matrix conjugated with 0.4mg/mL Streptavidin (GE Healthcare) was washed in 50 mL PBS and added to a 5 mL polystyrene tube (BD Falcon™). Biotinylated chemokine (1µM) was added to the tube and incubated for 20 min at RT to enable immobilization of the chemokine on the matrix via the biotin-streptavidin interaction. Next, the cells were added to the chemokine-matrix and incubated for 20 min at RT. The cells that did not bind to the matrix were removed by washing the matrix with PBS in a sterile 40um nylon filter (BD Falcon™ Cell Strainer). The flow through cells were stained with antibodies (Table 3), analysed by flow cytometry and compared with cells from peripheral blood that had not been incubated with the chemokine-matrix.

### Results and Discussion

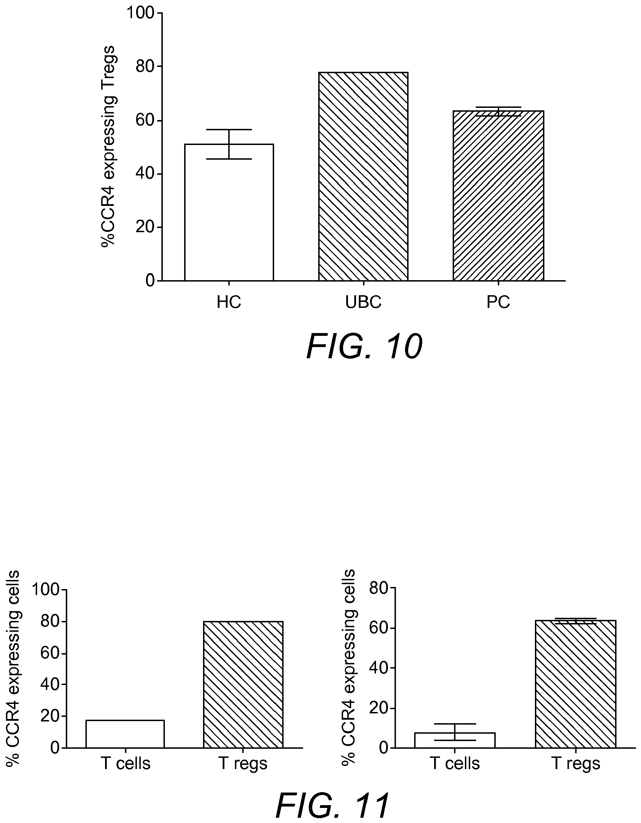

T regulatory cells (Tregs) are a subpopulation of T cells that suppress the immune system in order to maintain immune homeostasis. In cancer, the Tregs can inhibit an effective immune response against the tumor and thus removal of the Tregs could lead to a better immune activation against the cancer cells. White blood cells from patients with Urinary Bladder Cancer (UBC) and Pancreas Cancer (PC) were analyzed for the expression of chemokine receptors with flow cytometry. The cancer patients exhibited an increased frequency of circulating T regulatory cells (Tregs) that expressed the chemokine receptor CCR4, based upon flow cytometry data and binding by an anti-CCR4 antibody (Fig 10).

In both UBC and PC patients, CCR4 was highly upregulated on Tregs compared with the total T cell population (Fig 11).

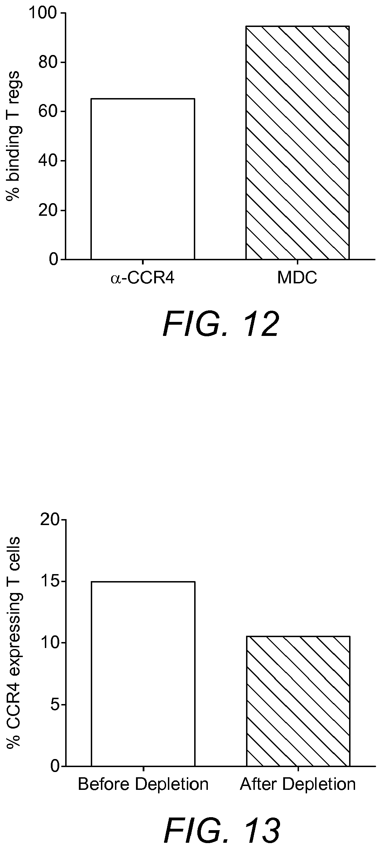

The ligand for CCR4 is the chemokine MDC (CCL22). In accordance with the CCR4 expression, the Tregs bound to biotinylated MDC (bMDC) (Fig 12).

The CCR4 expressing T cells could be depleted with bMDC-conjugated Sepharose Streptavidin Matrix (Fig 13).

We conclude that the frequency of Tregs that express CCR4 is enhanced in PC and UBC. These cells can bind the ligand bMDC. The Tregs express significantly more CCR4 than conventional T cells and can thus be specifically deleted with Sepharose Streptavidin matrix conjugated with bMDC.

### REFERENCE EXAMPLES 3 to 10:

### GENERAL PROTOCOLS - SYNTHESIS OF CHEMOKINES

### Assembly:

Chemical synthesis of chemokines was performed using standard Fmoc solid phase peptides synthesis (SPPS) techniques on an ABI 433 peptide synthesiser. DIC (0.5 M in DMF) and OxymaPure (0.5 M in DMF) were used for activation, acetic anhydride (0.5 M in DMF) for capping, and 20 % piperidine in DMF for Fmoc deprotection. Rink Amide resin was utilised for the generation of C-terminal amide chemokines and Wang resin for C-terminal acid chemokines. After assembly, the resin was washed with DMF and DCM and then dried *in vacuo.*

### Removal of Dde Protection:

The Dde protecting group was removed by treatment of resin with a solution of 2.5% hydrazine in DMF (200ml) over a 2 hour period. The resin was then washed with DMF.

### Labelling steps:

### 1. Couple Fmoc-8-amino-3,6-dioctanoic acid (PEG)

Resin was swollen in DMF and then a solution of Fmoc-8-amino-3,6-dioctanoic acid (0.38g, 1mmol), DIC solution (2ml, 0.5 M in DMF) and OxymaPure solution (2ml, 0.5 M in DMF) was added. The mixture was sonicated for 3 hours and then washed with DMF.

### 2. Capping

The resin was capped with acetic anhydride solution (0.5 M in DMF, 10ml) for 5 minutes and then washed with DMF.

### 3. Fmoc deprotection

Fmoc deprotection was carried out by treatment with 20% piperidine in DMF solution (2 x 50ml) for 15 minutes each. The resin was washed with DMF.

### 4. Couple Biotin-OSu

A solution of Biotin-OSu (341mg, 1mmol) and DIPEA (348µl) in DMF (10ml) was added to the resin and the mixture was sonicated for 3 hours. The resin was washed thoroughly with DMF and DCM then dried *in vacuo.*

### Cleavage:

Dry resin was treated with TFA (10 ml) containing a scavenger cocktail consisting of TIS (500 µl), thioanisole (500 µl), water (500 µl), DMS (500 µl), EDT (250 µl), NH₄I (500 µg) and phenol (500 µg) and the mixture was stirred at room temperature for 5 hours. The solution was filtered into cold ether and the resin rinsed with TFA. The precipitated peptide was centrifuged, washed with ether, centrifuged and lyophilised.

### Purification Protocol:

The crude peptide was purified by reverse phase HPLC (RP-HPLC) using a Jupiter C18, 250 x 21 mm column, 9 ml/min, eluting with an optimised gradient [Buffer A: water containing 0.1% TFA, Buffer B: acetonitrile containing 0.1% TFA].

### Folding Protocol:

Pure peptide (10mg) was dissolved into 6M GnHCI (16 ml) and then rapidly diluted to 2M GnHCI concentration by the addition of 50mM TRIS pH 8.5 (84 ml) containing 0.3mM GSSG and 3mM GSH. The mixture was stirred at room temperature for 24 hours and then analysed by RP-HPLC (Jupiter C18, 250 x 4.6 mm column, 10-60% B over 30 minutes). Purification by RP-HPLC using an optimised gradient afforded the desired product.

### REFERENCE Example 3 - biotinMCP-2 (CCL8)

Target Molecule: MCP-2 derivatised at the ε-amino side chain functionality of Lys(75) with PEG-Biotin (TFA salt)

Modifications: Human MCP-2 corresponding to residues 1-76, is initially expressed as 99 amino acids comprising the chemokine fold, and a 23 amino acid signal peptide which is cleaved off. The Gln at the N-terminus of the protein is subject to pyroGlu formation under physiological conditions. Thus Gln1 of the sequence was substituted with pyroglutamine to prevent mixed species of N-terminal Gln and pyroGlu being generated. This improves the yield of synthesis and ensures a homogeneous chemokine preparation through column manufacture and use. The naturally occurring lysine at position 75 was modified through biotinylation on the resin. A PEG spacer was incorporated between the ε-amino functionality and the biotin.

The linear amino acid sequence (SEQ ID NO: 1) is shown, prior to attachment of the PEG spacer and biotin molecules at amino acid 75 (K):
X1 = pyroGlu or Gln
X75 = an amino acid residue that can be biotinylated, such as lysine, ornithine or diaminopropionic acid and optionally is biotinylated, optionally via a spacer molecule such as PEG, in particular K(PEG-Biotin)

The engineered MCP-2 sequence was assembled on a solid support (Rink Amide resin), using Fmoc protocols for solid-phase peptide synthesis as described in the general protocols section:
X1 = pyroGlu or Gln
X75 = K(ivDde)

FmocLys(ivDde)-OH was incorporated as residue 75 to facilitate site-specific labelling at this position of the protein (SEQ ID NO: 2). Subsequent removal of the ivDde protecting group, followed by coupling of the PEG spacer and Biotin, was carried out as described in the general protocol section. Cleavage, purification and folding protocols were carried out as described to furnish the desired active chemokine (SEQ ID NO: 3).
X1 = pyroGlu or Gln
X75 = K(PEG-Biotin)

Electrospray ionisation with tandem mass spectrometry (ESI-TOF-MS) data of purified folded biotinMCP-2: obtained = 9263.6 Da; expected 9263.8 Da.

### Functional Assay Data:

biotinMCP-2 was tested for activity in an Aequorin assay against hCCR2b, (Euroscreen) and was shown to be a partial agonist with an EC50 value of 50.9nM. c.f. EC50 for recombinant native MCP-2 is 23.5nM (partial agonist).

### REFERENCE Example 4 - biotinRANTES (CCL5)

Target Molecule: RANTES derivatised at the ε-amino side chain functionality of Lys(67) with Biotin (TFA salt)

Modifications: Human RANTES corresponding to residues 1-68, is initially expressed as 91 amino acids comprising the chemokine fold, and a 23 amino acid signal peptide which is cleaved off. The single methionine (Met67) within the sequence was mutated to lysine, to mitigate against oxidation of this residue during the chain assembly, which was observed during the synthesis of the natural sequence derivative. This Met to Lys substitution provided a lysine at position 67 which was modified through biotinylation on the resin.

The linear amino acid sequence (SEQ ID NO: 4) is shown, prior to attachment of the biotin molecule at amino acid 67 (K):

The engineered RANTES sequence was assembled on a solid support (Wang resin), using Fmoc protocols for solid-phase peptide synthesis as described in the general protocols section: X= K(ivDde)

FmocLys(ivDde)-OH was incorporated as residue 67 to facilitate site-specific labelling at this position of the protein (SEQ ID NO: 5). Subsequent removal of the ivDde protecting group, followed by coupling of the Biotin, was carried out as described in the general protocol section. Cleavage, purification and folding protocols were carried out as described to furnish the desired active chemokine (SEQ ID NO: 6). X is an amino acid residue that can be biotinylated, such as lysine, ornithine or diaminopropionic acid and optionally is biotinylated (e.g. K-biotin), optionally via a spacer molecule such as PEG, in particular K(PEG-Biotin)

Electrospray ionisation with tandem mass spectrometry (ESI-TOF-MS) data of purified folded biotinRANTES: obtained = 8068.9 Da; expected 8070.2 Da.

### Functional Assay Data:

BiotinRANTES was tested for agonist activity in an Aequorin assay against hCCR5, (Euroscreen) and an EC50 value of 0.5nM was reported.

### REFERENCE Example 5 - biotinMIP-3α (CCL20)

Target Molecule: MIP-3α derivatised at the ε-amino side chain functionality of Lys(68) with PEG-Biotin (TFA salt)

Modifications: Human MIP-3α corresponding to residues 1-70, is initially expressed as 96 amino acids comprising the chemokine fold, and a 26 amino acid signal peptide which is cleaved off. The naturally occurring lysine at position 68 was modified through biotinylation on the resin. A PEG spacer was incorporated between the ε-amino functionality and the biotin.

The linear amino acid sequence (SEQ ID NO: 7) is shown, prior to attachment of the PEG spacer and biotin molecules at amino acid 68 (K):

The engineered MIP-3α sequence was assembled on a solid support (Wang resin), using Fmoc protocols for solid-phase peptide synthesis as described in the general protocols section: X= K(ivDde)

FmocLys(ivDde)-OH was incorporated as residue 68 to facilitate site-specific labelling at this position of the protein (SEQ ID NO: 8). Subsequent removal of the ivDde protecting group, followed by coupling of the PEG spacer and Biotin, was carried out as described in the general protocol section. Cleavage, purification and folding protocols were carried out as described to furnish the desired active chemokine (SEQ ID NO: 9). X is an amino acid residue that can be biotinylated, such as lysine, ornithine or diaminopropionic acid and optionally is biotinylated, optionally via a spacer molecule such as PEG, in particular K(PEG-Biotin)

Electrospray ionisation with tandem mass spectrometry (ESI-TOF-MS) data of purified folded biotinMip-3α: obtained = 8396.4 Da; expected 8397.0 Da.

### Functional Assay Data:

BiotinMIP-3α was tested for agonist activity in an Aequorin assay against hCCR6, (Euroscreen) and an EC50 value of 1.6nM was reported. c.f. EC50 for recombinant native MIP-3α is 1.0nM.

### REFERENCE Example 6 - biotinSDF-1α (CXCL12)

Target Molecule: SDF-1α derivatised at the ε-amino side chain functionality of Lys(64) with Biotin (TFA salt)

Modifications: Truncated form of human SDF-1α corresponding to residues 1-67 of the mature protein, which encompasses the sequence corresponding to the chemokine fold. The full length mature protein is 72 amino acids (the signal peptide is 21 amino acids in a 93 amino acid immature protein). The naturally occurring lysine at position 64 was modified through biotinylation on the resin.

The linear amino acid sequence (SEQ ID NO: 10) is shown, prior to attachment of the biotin molecule at amino acid 64 (K):

The engineered SDF-1α sequence was assembled on a solid support (Wang resin), using Fmoc protocols for solid-phase peptide synthesis as described in the general protocols section: X= K(ivDde)

FmocLys(ivDde)-OH was incorporated as residue 64 to facilitate site-specific labelling at this position of the protein (SEQ ID NO: 11). Subsequent removal of the ivDde protecting group, followed by coupling of the Biotin, was carried out as described in the general protocol section. Cleavage, purification and folding protocols were carried out as described to furnish the desired active chemokine (SEQ ID NO: 12). X is an amino acid residue that can be biotinylated, such as lysine, ornithine or diaminopropionic acid and optionally is biotinylated, optionally via a spacer molecule such as PEG, especially K(Biotin)

Electrospray ionisation with tandem mass spectrometry (ESI-TOF-MS) data of purified folded biotinSDF-1α: obtained = 8055.5 Da; expected 8057.5 Da.

### Functional Assay Data:

biotinSDF-1α was tested for agonist activity in an Aequorin assay against hCXCR4, (Euroscreen) and an EC50 value of 17.3nM was reported. c.f. EC50 for recombinant native SDF-1α is 12.0nM.

### REFERENCE Example 7 - biotinMDC (CCL22)

Target Molecule: MDC derivatised at the ε-amino side chain functionality of Lys(66) with PEG-Biotin (TFA salt)

Modifications: Human MDC corresponding to residues 1-69, is initially expressed as 93 amino acids comprising the chemokine fold, and a 24 amino acid signal peptide which is cleaved off. The naturally occurring lysine at position 66 was modified through biotinylation on the resin. A PEG spacer was incorporated between the ε-amino functionality and the biotin.

The linear amino acid sequence (SEQ ID NO: 13) is shown, prior to attachment of the PEG spacer and biotin molecules at amino acid 66 (K):

The engineered MDC sequence was assembled on a solid support (Rink Amide resin), using Fmoc protocols for solid-phase peptide synthesis as described in the general protocols section: X= K(ivDde)

FmocLys(ivDde)-OH was incorporated as residue 66 to facilitate site-specific labelling at this position of the protein (SEQ ID NO: 14). Subsequent removal of the ivDde protecting group, followed by coupling of the PEG spacer and Biotin, was carried out as described in the general protocol section. Cleavage, purification and folding protocols were carried out as described to furnish the desired active chemokine (SEQ ID NO: 15). X is an amino acid residue that can be biotinylated, such as lysine, ornithine or diaminopropionic acid and optionally is biotinylated, optionally via a spacer molecule such as PEG, especially K(PEG-Biotin)

Electrospray ionisation with tandem mass spectrometry (ESI-TOF-MS) data of purified folded biotinMDC: obtained = 8456.1 Da; expected 8456.9 Da.

### Functional Assay Data:

BiotinMDC was tested for agonist activity in an Aequorin assay against hCCR4, (Euroscreen) and an EC50 value of 4.5nM was reported. c.f. EC50 for recombinant native MDC is 3.6nM.

### REFERENCE Example 8 - biotinTARC (CCL17)

Target Molecule: TARC derivatised at the ε-amino side chain functionality of Lys(72) with PEG-Biotin (TFA salt)

Modifications: Human TARC corresponding to residues 1-71, is initially expressed as 94 amino acids comprising the chemokine fold, and a 23 amino acid signal peptide which is cleaved off. An additional lysine was inserted at the C-terminus, at position 72, and modified through biotinylation on the resin. A PEG spacer was incorporated between the ε-amino functionality and the biotin.

The linear amino acid sequence (SEQ ID NO: 16) is shown, prior to attachment of the PEG spacer and biotin molecules at amino acid 72 (K): X is an amino acid residue that can be biotinylated, such as lysine, ornithine or diaminopropionic acid and optionally is biotinylated (e.g. K-biotin), optionally via a spacer molecule such as PEG, in particular K(PEG-Biotin)

The engineered TARC sequence was assembled on a solid support (Rink Amide resin), using Fmoc protocols for solid-phase peptide synthesis as described in the general protocols section: X= K(ivDde)

FmocLys(ivDde)-OH was incorporated as residue 72 to facilitate site-specific labelling at this position of the protein (SEQ ID NO: 17). Subsequent removal of the ivDde protecting group, followed by coupling of the PEG spacer and Biotin, was carried out as described in the general protocol section. Cleavage, purification and folding protocols were carried out as described to furnish the desired active chemokine (SEQ ID NO: 18). X = K(PEG-Biotin)

Electrospray ionisation with tandem mass spectrometry (ESI-TOF-MS) data of purified folded biotinTARC: obtained = 8577.2 Da; expected 8577.8 Da.

### Functional Assay Data:

BiotinTARC was tested for agonist activity in an Aequorin assay against hCCR4, (Euroscreen) and an EC50 value of 3.1nM was reported. c.f. EC50 for recombinant native TARC is 2.6nM.

### Example 9 - biotinMIP-3β (CCL19)

Target Molecule: MIP-3β derivatised at the ε-amino side chain functionality of Lys(78) with Biotin (TFA salt)

Modifications: Human MIP-3β corresponding to residues 1-77, is initially expressed as 98 amino acids comprising the chemokine fold, and a 21 amino acid signal peptide which is cleaved off. An additional lysine was inserted at the C-terminus, at position 78, and modified through biotinylation on the resin.

The linear amino acid sequence (SEQ ID NO: 19) is shown, prior to attachment of the biotin molecule at amino acid 78 (K): X is an amino acid residue that can be biotinylated, such as lysine, ornithine or diaminopropionic acid and optionally is biotinylated (e.g. K-biotin), optionally via a spacer molecule such as PEG, in particular K(PEG-Biotin)

The engineered MIP-3β sequence was assembled on a solid support (Rink Amide resin), using Fmoc protocols for solid-phase peptide synthesis as described in the general protocols section: X is FmocLys(ivDde)

FmocLys(ivDde)-OH was incorporated as residue 78 to facilitate site-specific labelling at this position of the protein (SEQ ID NO: 20). Subsequent removal of the ivDde protecting group, followed by coupling of the Biotin, was carried out as described in the general protocol section. Cleavage, purification and folding protocols were carried out as described to furnish the desired active chemokine (SEQ ID NO: 21). X is K(Biotin)

Electrospray ionisation with tandem mass spectrometry (ESI-TOF-MS) data of purified folded biotinMIP-3β: obtained = 9148.8 Da; expected 9149.7 Da.

### Functional Assay Data:

biotinMip-3β was tested for agonist activity in an Aequorin assay against hCCR7, (Euroscreen) and an EC50 value of 11.0nM was reported. c.f. EC50 for recombinant native MIP-3β is 1.6nM.

### REFERENCE Example 10 - biotinITAC (CXCL11)

Target Molecule: ITAC derivatised with Biotin at the ε-amino side chain functionality of an additional Lysine inserted at the C-terminus after a PEG spacer (TFA salt)

Modifications: Human ITAC corresponding to residues 1-73, is initially expressed as 94 amino acids comprising the chemokine fold, and a 21 amino acid signal peptide which is cleaved off. A PEG spacer and an additional lysine were inserted at the C-terminus, and modified through biotinylation on the resin. The PEG spacer was incorporated at the C-terminus between the protein and the additional lysine.

The linear amino acid sequence (SEQ ID NO: 22) is shown, prior to attachment of the PEG spacer, additional lysine and biotin molecules:

The engineered ITAC sequence was assembled on a solid support (Wang resin), using Fmoc protocols for solid-phase peptide synthesis as described in the general protocols section: X is PEG-K(ivDde)

Fmoc-12-amino-4,7,10-trioxadodecanoic acid followed by FmocLys(ivDde)-OH were incorporated at the C-terminus to facilitate site-specific labelling with biotin at the ε-amino side chain functionality of the additional Lys (SEQ ID NO: 24). Subsequent removal of the ivDde protecting group, followed by coupling of the Biotin, was carried out as described in the general protocol section. Cleavage, purification and folding protocols were carried out as described to furnish the desired active chemokine (SEQ ID NO: 25). X is PEG-K(Biotin)

Electrospray ionisation with tandem mass spectrometry (ESI-TOF-MS) data of purified folded biotinITAC: obtained = 8866.5 Da; expected 8860.6 Da.

### Functional Assay Data:

biotinITAC was tested for agonist activity in an Aequorin assay against hCXCR3, (Euroscreen) and an EC50 value of 15.7nM was reported. c.f. EC50 for recombinant native ITAC is 0.7nM.

### REFERENCE Example 11 - biotinTECK (CCL25)

Target Molecule: TECK (Met to Nleu substitution) derivatised at the ε-amino side chain functionality of Lys72 with PEG-Biotin (TFA salt)

Modifications: Truncated form of human TECK corresponding to residues 1-74 of the mature protein, which encompasses the sequence corresponding to the chemokine fold. The full length mature protein is 127 amino acids (the signal peptide is 23 amino acids in a 150 amino acid immature protein). The single methionine within the sequence was altered to Norleucine, to mitigate against oxidation of this residue during the chain assembly, which was observed during the synthesis of the natural sequence derivative. The Gln at the N-terminus of the proteins is subject to pyroGlu formation under physiological conditions. Thus Gln1 of the sequence was substituted with pyroglutamine to prevent mixed species of N-terminal Gln and pyroGlu being generated. This improves the yield of synthesis and ensures a homogeneous chemokine preparation through column manufacture and use. The naturally occurring lysine at position 72 was modified through biotinylation on the resin. A PEG spacer was incorporated between the ε-amino functionality and the biotin.

The linear amino acid sequence (SEQ ID NO: 26) is shown, prior to attachment of the PEG spacer and biotin molecules at amino acid 72 (K):
X1 = pyroGlu or Gln
X64 = Norleucine
X72 = an amino acid residue that can be biotinylated, such as lysine, ornithine or diaminopropionic acid and optionally is biotinylated, optionally via a spacer molecule such as PEG, e.g. K(PEG-Biotin)

The engineered TECK sequence was assembled on a solid support (Wang resin), using Fmoc protocols for solid-phase peptide synthesis as described in the general protocols section:
X1 = pyroGlu or Gln
X64 = Norleucine
X72 = K(ivDde)

FmocLys(ivDde)-OH was incorporated as residue 72 to facilitate site-specific labelling at this position of the protein (SEQ ID NO: 27). Subsequent removal of the ivDde protecting group, followed by coupling of the PEG spacer and Biotin, was carried out as described in the general protocol section. Cleavage, purification and folding protocols were carried out as described to furnish the desired active chemokine (SEQ ID NO: 28).
X1 = pyroGlu or Gln
X64 = Norleucine
X72 is K(PEG-Biotin)

Electrospray ionisation with tandem mass spectrometry (ESI-TOF-MS) data of purified folded biotinTECK(Met to Nleu substitution): obtained = 8958.5 Da; expected 8959.6 Da.

### Functional Assay Data:

biotinTECK(Met to Nleu substitution) was tested for agonist activity in an Aequorin assay against hCCR9, (Euroscreen) and an EC50 value of 63.6nM was reported. c.f. EC50 for recombinant native TECK is 67.9nM.

### REFERENCE Example 12 - biotinCTAC (CCL27)

Target Molecule: CTAC derivatised at the ε-amino side chain functionality of Lys(87) with PEG-Biotin (TFA salt)

Modifications: Human CTAC corresponding to residues 1-88, is initially expressed as 112 amino acids comprising the chemokine fold, and a 24 amino acid signal peptide which is cleaved off. The Met(87) within the sequence was mutated to lysine to provide a lysine at position 87 which was modified through biotinylation on the resin. A PEG spacer was incorporated between the ε-amino functionality and the biotin.

The linear amino acid sequence (SEQ ID NO: 29) is shown, prior to attachment of the PEG spacer and biotin molecules: X is an amino acid residue that can be biotinylated, such as lysine, ornithine or diaminopropionic acid and optionally is biotinylated, optionally via a spacer molecule such as PEG, e.g. K(PEG-Biotin)

The engineered CTAC sequence was assembled on a solid support (Wang resin), using Fmoc protocols for solid-phase peptide synthesis as described in the general protocols section: X = K(ivDde)

FmocLys(ivDde)-OH was incorporated as residue 87 to facilitate site-specific labelling at this position of the protein (SEQ ID NO: 30). Subsequent removal of the ivDde protecting group, followed by coupling of the PEG spacer and Biotin, was carried out as described in the general protocol section. Cleavage, purification and folding protocols were carried out as described to furnish the desired active chemokine (SEQ ID NO: 31). X = K(PEG-Biotin)

Electrospray ionisation with tandem mass spectrometry (ESI-TOF-MS) data of purified folded biotinCTAC: obtained = 10513.4 Da; expected 10514.2Da.

### Functional Assay Data:

BiotinCTAC was tested for agonist activity in an Aequorin assay against hCCR10, (Euroscreen) and an EC50 value of 49.4nM was reported. c.f. EC50 for recombinant native CTAC is 33.5nM.

### REFERENCE EXAMPLE 13 - biotinIP-10 (CXCL10)

Target Molecule: IP-10 derivatised with Biotin at the ε-amino side chain functionality of an additional Lysine inserted at the C-terminus after a PEG spacer (TFA salt)

Modifications: Human IP-10 corresponding to residues 1-77, is initially expressed as 98 amino acids comprising the chemokine fold, and a 21 amino acid signal peptide which is cleaved off. A PEG spacer and an additional lysine were inserted at the C-terminus, and modified through biotinylation on the resin. The PEG spacer was incorporated at the C-terminus between the protein and the additional lysine.

The linear amino acid sequence (SEQ ID NO: 32) is shown, prior to attachment of the PEG spacer, additional lysine and biotin molecules:

The engineered IP-10 sequence was assembled on a solid support (Wang resin), using Fmoc protocols for solid-phase peptide synthesis as described in the general protocols section: X is K(ivDde), optionally attached via a spacer such as PEG, e.g. -PEG-K(ivDde)

Fmoc-8-amino-3,6-dioctanoic acid followed by FmocLys(ivDde)-OH were incorporated at the C-terminus to facilitate site-specific labelling with biotin at the ε-amino side chain functionality of the additional Lys (SEQ ID NO: 33). Subsequent removal of the ivDde protecting group, followed by coupling of the Biotin, was carried out as described in the general protocol section. Cleavage, purification and folding protocols were carried out as described to furnish the desired active chemokine. The final active chemokine thus has the following sequence (SEQ ID NO: 34): X is an amino acid residue that can be biotinylated, such as lysine, ornithine or diaminopropionic acid and optionally is biotinylated, optionally via a spacer molecule such as PEG, e.g. K(PEG-Biotin) and may be attached via a spacer molecule, e.g.PEG-K(Biotin)

Electrospray ionisation with tandem mass spectrometry (ESI-TOF-MS) data of purified folded biotinIP-10: obtained = 9141.0 Da; expected 9141.9 Da.

### Functional Assay Data:

BiotinlP-10 was tested for agonist activity in an Aequorin assay against hCXCR3, (Euroscreen) and an EC50 value of 8.7nM was reported. c.f. EC50 for recombinant native IP-10 is 4.4nM.
## Claims

1. A binding reagent capable of specifically binding to the chemokine receptor, CCR7, for use in the treatment of cancer, wherein the binding reagent is immobilized on a solid support contained within an apheresis column, to which is applied peripheral blood from a patient thus removing CCR7 expressing cells from the peripheral blood of the patient, wherein the binding reagent is a chemokine is selected from CCL19 or CCL21.

2. The binding reagent for use according to claim 1 wherein:
(i) the cancer treatment serves to reduce the levels of circulating tumour cells; and/or
(ii) the cancer treatment serves to reduce the incidence of tumour metastasis; and/or
(iii) the cancer is a leukaemia, optionally wherein the leukemia is selected from chronic lymphocytic leukaemia chronic myeloid leukemia, AML, ALL and leukemic phase of lymphoma;
and/or
(iv) the binding reagent capable of specifically binding to CCR7 is an agonist or an antagonist of the CCR7;
and/or
(v) the binding reagent capable of specifically binding to CCR7 is selected from an antibody and a chemokine.

3. The binding reagent for use according to any one of claims 1 to 2(i)-(iii) wherein the cancer is PC or UBC or wherein the CCR7 expressing cells are selected from tumour cells, central memory T lymphocytes and regulatory lymphocytes.

4. The binding reagent for use according to any one of claims 1 to 3 wherein the patient has been selected for treatment on the basis of detecting an increase in the level of CCR7 expressing cells, levels of expression of CCR7, and/or levels of cells with high expression of CCR7 in a sample obtained from the patient, optionally wherein 20-90% of the patient's blood is applied to the column in a single treatment.

5. A binding reagent capable of specifically binding to one or more regulatory T cell receptors, in particular the cutaneous lymphocyte antigen (CLA) receptor, for use in a method for treating cancer wherein the binding reagent is immobilized directly or indirectly on a solid support contained within an apheresis column, to which is applied peripheral blood from a patient thus removing one or more regulatory T cell receptor-expressing cells (Tregs) from the peripheral blood of the patient and wherein the binding reagent is capable of binding to CCR7 wherein the binding reagent is a chemokine is selected from CCL19 or CCL21.

## Patentansprüche

1. Bindungsreagenz, welches spezifisch an den Chemokinrezeptor, CCR7, zu binden vermag, zur Verwendung bei der Behandlung von Krebs, wobei das Bindungsreagenz auf einem innerhalb einer Apheresesäule enthaltenen festen Träger immobilisiert ist, auf welche peripheres Blut von einem Patienten aufgetragen ist, wodurch CCR7-exprimierende Zellen aus dem peripheren Blut des Patienten entfernt werden, wobei das Bindungsreagenz ein aus CCL19 oder CCL21 ausgewähltes Chemokin ist.

2. Bindungsreagenz zur Verwendung nach Anspruch 1, wobei:
(i) die Krebsbehandlung zur Verringerung der Spiegel von zirkulierenden Tumorzellen dient;
und/oder
(ii) die Krebsbehandlung zur Verringerung des Auftretens von Tumormetastase dient;
und/oder
(iii) der Krebs eine Leukämie ist, optional wobei die Leukämie aus chronischer lymphatischer Leukämie, chronischer myeloischer Leukämie, AML, ALL und leukämischer Phase des Lymphoms ausgewählt ist;
und/oder
(iv) das Bindungsreagenz, das spezifisch an CCR7 zu binden vermag, ein Agonist oder ein Antagonist von CCR7 ist;
und/oder
(v) das Bindungsreagenz, das spezifisch an CCR7 zu binden vermag, aus einem Antikörper und einem Chemokin ausgewählt ist.

3. Bindungsreagenz zur Verwendung nach einem der Ansprüche 1 bis 2(i)-(iii), wobei der Krebs PC oder UBC ist, oder wobei die CCR7-exprimierenden Zellen aus Tumorzellen, zentralen Gedächtnis-T-Lymphozyten und regulatorischen Lymphozyten ausgewählt sind.

4. Bindungsreagenz zur Verwendung nach einem der Ansprüche 1 bis 3, wobei der Patient zur Behandlung auf der Grundlage des Nachweises eines Anstiegs des Spiegels von CCR7-exprimierenden Zellen, der Spiegel der Expression von CCR7 und/oder der Spiegel von Zellen mit hoher Expression von CCR7 in einer vom Patienten erhaltenen Probe ausgewählt wurde, optional wobei in einer einzelnen Behandlung 20-90 % des Blutes des Patienten auf die Säule aufgetragen werden.

5. Bindungsreagenz, welches spezifisch an einen oder mehrere regulatorische T-Zell-Rezeptoren, insbesondere den Rezeptor für das kutane Lymphozyten-Antigen (CLA), zu binden vermag, zur Verwendung in einem Verfahren zur Behandlung von Krebs, wobei das Bindungsreagenz direkt oder indirekt auf einem in einer Apheresesäule enthaltenen festen Träger immobilisiert ist, auf welche peripheres Blut von einem Patienten aufgetragen ist, wodurch eine oder mehrere regulatorische T-Zellrezeptor-exprimierende Zellen (Tregs) aus dem peripheren Blut des Patienten entfernt werden, und wobei das Bindungsreagenz an CCR7 zu binden vermag, wobei das Bindungsreagenz ein aus CCL19 oder CCL21 ausgewähltes Chemokin ist.

## Revendications

1. Réactif de liaison capable de se lier spécifiquement à un récepteur de chimiokine CCR7 à utiliser dans le traitement du cancer, ledit réactif de liaison étant immobilisé sur un support solide contenu dans une colonne d'aphérèse, à laquelle est appliqué le sang périphérique d'un patient, éliminant ainsi des cellules exprimant CCR7 du sang périphérique du patient, ledit réactif de liaison étant une chimiokine choisie parmi CCL19 ou CCL21.

2. Réactif de liaison pour l'usage selon la revendication 1, dans lequel:
(i) le traitement du cancer sert à réduire les niveaux de cellules tumorales circulantes ;
et / ou
(ii) le traitement du cancer sert à réduire l'incidence de métastases tumorales ;
et / ou
(iii) le cancer est une leucémie, éventuellement dans lequel la leucémie est choisie parmi la leucémie lymphoïde chronique, la leucémie myéloïde chronique, la LMA, la LAL et la phase leucémique du lymphome ;
et / ou
(iv) le réactif de liaison capable de se lier spécifiquement à CCR7 est un agoniste ou un antagoniste de CCR7 ;
et / ou
(v) le réactif de liaison capable de se lier spécifiquement à CCR7 est choisi parmi un anticorps et une chimiokine.

3. Réactif de liaison pour l'usage selon l'une quelconque des revendications 1 à 2(i)-(iii) dans lequel le cancer est PC ou UBC, ou dans lequel les cellules exprimant CCR7 sont sélectionnés des cellules tumorales, des lymphocytes T à mémoire centrale et des lymphocytes régulateurs.

4. Réactif de liaison pour l'usage selon l'une quelconque des revendications 1 à 3, dans lequel le patient a été sélectionné pour le traitement sur la base de la détection d'une augmentation du niveau des cellules exprimant CCR7, des niveaux d'expression de CCR7 et/ou des niveaux de cellules avec une expression élevée de CCR7 dans un échantillon obtenu à partir du patient, éventuellement dans lequel environ 20 à 90% du sang du patient est appliqué à la colonne en un seul traitement.

5. Réactif de liaison capable de se lier spécifiquement à un ou plusieurs récepteurs régulateurs des cellules T, en particulier le récepteur de l'antigène cutané des lymphocytes (CLA), à utiliser dans un procédé pour le traitement du cancer, dans lequel le réactif de liaison est immobilisé directement ou indirectement sur un support solide contenu dans une colonne d'aphérèse, à laquelle est appliqué le sang périphérique d'un patient, éliminant ainsi une ou plusieurs cellules régulatrices exprimant le récepteur des cellules T (Tregs) du sang périphérique du patient, et dans lequel le réactif de liaison est capable de se lier à CCR7, dans lequel le réactif de liaison est une chimiokine choisie parmi CCL19 ou CCL21.
